# EUROPEAN PATENT SPECIFICATION

(11) **EP 1 691 823 B1**
(45) Date of publication and mention of the grant of the patent: **10.10.2012**
(21) Application number: 04801605.9
(22) Date of filing: 09.12.2004
(51) Int. Cl.: A61K 38/02, A61K 38/28, A61K 38/39, C07K 2/00, C07K 14/62, C07K 14/78, A61N 1/00, A61N 1/30

(54) **TRANSDERMAL SYSTEM FOR SUSTAINED DELIVERY OF POLYPEPTIDES**
TRANSDERMALES SYSTEM FÜR DIE ANHALTENDE ABGABE VON POLYPEPTIDEN
SYSTEME TRANSDERMIQUE POUR ADMINISTRATION PROLONGEE DE POLYPEPTIDES

(30) Priority: 09.12.2003 IL 15927303
(43) Date of publication of application: 23.08.2006
(73) Proprietor: Syneron Medical Ltd., 20692 Yoqneam Illit (IL)
(72) Inventor: LEVIN, Galit, 42954 Nordiya (IL); SACKS, Hagit, 71700 Modi'in (IL)
(74) Representative: Becker Kurig Straus
(86) International application number: PCT/IL2004/001119
(87) International publication number: WO 2005/056075

(56) References cited:
- US-A- 5 087 240
- US-A- 5 418 222
- US-A- 5 543 098
- US-A- 5 961 482
- US-A1- 2003 143 274
- US-B2- 6 490 482
- SINTOV A C ET AL: "Radiofrequency-driven skin microchanneling as a new way for electrically assisted transdermal delivery of hydrophilic drugs" JOURNAL OF CONTROLLED RELEASE, ELSEVIER, AMSTERDAM, NL, vol. 89, no. 2, 29 April 2003 (2003-04-29), pages 311-320, XP004421365 ISSN: 0168-3659
- [Online] 01 November 2005, XP002987518 Retrieved from the Internet: <URL:http://hyperphysics.phy-astr.gsu.edu/e ms2.html>

## Description

### FIELD OF THE INVENTION

The present invention relates to a transdermal system for sustained delivery of high molecular weight hydrophilic drugs, especially peptide-, polypeptide- or protein-drugs, and to methods of use thereof. The system comprises an apparatus that generates micro-channels in the skin of a subject in conjunction with a transdermal patch comprising at least one drug reservoir layer comprising a polymeric matrix containing the peptide-, polypeptide- or protein-drug.

### BACKGROUND OF THE INVENTION

The delivery of drugs through the skin provides many advantages. Primarily, such a means of delivery is a comfortable, convenient and noninvasive way of administering drugs. The variable rates of absorption and metabolism encountered in oral treatment are avoided, and other inherent inconveniences, e.g., gastrointestinal irritation and degradation of certain drugs via gastrointestinal enzymes, are eliminated. Transdermal drug delivery theoretically enables a high degree of control over blood concentrations of any particular drug. In reality, many problems remain to be resolved in order to achieve these advantages.

Skin is a structurally complex, relatively thick membrane. Molecules moving from the environment into and through intact skin must first penetrate the stratum corneum. They must then penetrate the viable epidermis, the papillary dermis, and the capillary walls into the blood stream or lymph channels. To be so absorbed, molecules must overcome a different resistance to penetration in each type of tissue. Transport across the skin membrane is thus a complex phenomenon. However, it is the cells of the stratum corneum, which present the primary barrier to transdermally administered drugs. The stratum corneum is a thin layer of dense, highly keratinized cells approximately 10-30 microns thick over most of the body. It is believed that the high degree of keratinization within these cells and their dense packing create a substantially impermeable barrier to drug penetration. With many drugs, the rate of permeation through the skin is extremely low and is particularly problematic for high molecular weight drugs such as polypeptides and proteins. Consequently, a means for enhancing the permeability of the skin is desired to effect transport of the drug into and through intact skin.

In order to increase the rate at which a drug penetrates through the skin, various approaches have been adapted, each of which involves the use of either a physical penetration enhancer or a chemical penetration enhancer. Physical enhancement of skin permeation includes, for example, electrophoretic techniques such as iontophoresis or electroporation. The use of ultrasound (or "sonophoresis") as a physical penetration enhancer has also been studied. Chemical enhancers are compounds that are administered along with the drug (or in some cases the skin may be pretreated with a chemical enhancer) in order to increase the permeability of the stratum corneum, and thereby provide for enhanced penetration of the drug through the skin. However, a major disadvantage exists when using such chemical enhancers as skin damage, irritation, and sensitization are often encountered.

U.S. Pat. No. 6,148,232 to Avrahami, which is incorporated herein by reference, describes a device for ablating the stratum corneum of a subject. The device includes a plurality of electrodes, which are applied at respective points on skin of a subject. A power source applies electrical energy between two or more of the electrodes to cause ablation of distinct regions of the stratum corneum (SC), primarily beneath the respective electrodes, and to generate micro-channels. Various techniques for limiting ablation to the stratum corneum are described, including spacing of the electrodes and monitoring the electrical resistance of skin between adjacent electrodes. U.S. Pat. Nos. 6,597,946; 6,611,706; 6,708,060; and 6,711,435 to Avrahami, all assigned to the applicant of the present application and incorporated by reference as if fully set forth herein, disclose additional devices for ablating the stratum corneum and generating micro-channels so as to facilitate transdermal passage of substances through the skin. The devices are aimed at reducing sensation and minimizing damage to skin underlying the stratum corneum during micro-channel generation.

U.S. Pat. No. 6,490,482 discloses an electroporation device which creates aqueous pathways in lipid bilayer membranes. According to US 6,490,482, a contact electrode pair for electroporation is affixed to an agent permeable membrane such that a device of membrane electrode is formed. Electrode and agent permeable membrane are present as one entity. The active agent can be either water-soluble or lipid soluble, and the membrane can be either hydrophilic or hydrophobic membrane. The pharmaceutical composition according to US 6,490,482 is present in a solution only. However, said patent does not disclose an array of electrodes and a patch adapted to be applied to the region of the skin where micro-channels are present after removal of the apparatus.

It has been long appreciated that administration of a therapeutic agent in a manner that does not afford controlled release may lead to substantial oscillation of its levels, at times reaching concentrations that could be toxic or produce undesirable side effects, and at other times falling below the levels required for therapeutic efficacy. A primary goal of the use of devices and/or methods for controlled release is to produce greater control over the systemic levels of therapeutic agents.

Various strategies have been developed aiming at achieving controlled release of a therapeutic agent. Release by controlled diffusion is one of these strategies. Different materials have been used to fabricate diffusion-controlled slow release devices. These materials include non-degradable polymers such as polydimethyl siloxane, ethylene-vinyl acetate copolymers, and hydroxylalkyl methacrylates as well as degradable polymers, among them lactic/glycolic acid copolymers. Microporous membranes fabricated from ethylene-vinyl acetate copolymers have been used for release of proteins, affording a high release capacity.

An additional strategy for controlled release involves chemically controlled sustained release, which requires chemical cleavage from a substrate to which a therapeutic agent is immobilized, and/or biodegradation of the polymer to which the agent is immobilized. This category also includes controlled non-covalent dissociation, which relates to release resulting from dissociation of an agent, which is temporarily bound to a substrate by non-covalent binding. This method is particularly well suited for controlled release of proteins or peptides, which are macromolecules capable of forming multiple non covalent, ionic, hydrophobic, and/or hydrogen bonds that afford stable but not permanent attachment of proteins to a suitable substrate.

U.S. Pat. No. 5,418,222 relates to single and multiple layer collagen films for use in controlled release of active ingredients, particularly of PDGF. U.S. Pat. No. 5,512,301 relates to collagen-containing sponges comprising an absorbable gelatin sponge, collagen, and an active ingredient. The collagen films disclosed in U.S. Pat. No. 5,418,222 and the collagen sponges disclosed in U.S. Pat. No. 5,512,301 provide a steady, continuous and sustained release of therapeutic agents over an extended period of time.

U.S. Pat. No. 5,681,568 discloses a device comprising a microporous underlayment with microcapillary pores wherein said pores are coated but not completely filled by a microskin to which a biologically active macromolecular agent is bound. Microporous underlayments comprise a polymer, and the microskin comprises substances selected from collagens, fibronectins, laminins, proteoglycans, and glycosaminoglycans. It is believed that such devices be useful for optimizing the delivery of macromolecules, particularly of growth factors, to a therapeutic target.

U.S. Pat. No. 6,596,293 discloses a method for preparing a drug delivery material and device comprising cross-linking of a biological polymer with a cross-linking agent and loading the cross-linked biopolymer with a bioactive agent.

U.S. Pat. No. 6,275,728 provides a thin film drug reservoir for an electrotransport drug delivery device comprising a hydratable, hydrophilic polymer, said film capable of forming a hydrogel when placed in contact with a hydrating liquid.

International Patent Application WO 2004/039428, assigned to the applicant of the present application, discloses a system for transdermal delivery of a dried pharmaceutical composition. The system comprises an apparatus for facilitating transdermal delivery of a drug through skin of a subject, which generates at least one micro-channel in an area on the skin of the subject, and a patch comprising a therapeutically effective amount of a dried pharmaceutical composition. WO 2004/039428 provides, for the first time, an efficient method for transdermal delivery of hydrophilic high molecular weight proteins.

There still remains an unmet need for devices and methods for transdermal delivery of hydrophilic high molecular weight medications, which achieve sustained and controlled delivery of such medications. The advantages of these devices and methods would be particularly striking for polypeptides and proteins as well as for other bioactive water-soluble drugs.

### SUMMARY OF THE INVENTION

The present invention provides a system for ablating the skin and applying to the pretreated skin an active agent, the system designed to achieve a slow and sustained delivery of the active agent into the systemic circulation.

The system comprises a patch comprising at least one drug reservoir layer to which the active agent is non-covalently bound and is releasable therefrom.

It should be appreciated that polymeric matrices are well known as substrates or implants for delivery of polypeptides or proteins into tissues or the blood circulation. However, nowhere in the background art is it disclosed nor suggested that a combination of a polymeric matrix and a hydrophilic high molecular weight polypeptide or protein can be used in a patch for transdermal delivery. It is now disclosed, for the first time, that use of a patch comprising a polymeric drug reservoir layer comprising a high molecular weight polypeptide as an active agent, when placed on an area of the skin pretreated by an apparatus that generates micro-channels, enables achieving therapeutically effective serum levels of the high molecular weight polypeptide for extended periods of time.

According to the invention a system for facilitating transdermal delivery of an active agent through skin of a subject is provided said system comprising:
(i) an apparatus comprising:
   a. an electrode cartridge comprising an array of electrodes; and
   b. a main unit comprising a control unit, which is adapted to apply electrical energy to the electrodes when said electrodes are in vicinity of the skin, enabling ablation of stratum corneum in an area beneath the electrodes, thereby generating a plurality of micro-channels extending from the surface of the skin through all or a significant part of the stratum corneum; and
(ii) a patch adapted to be applied to the area of the skin where micro-channels are present after removal of the apparatus, the patch comprising at least one drug reservoir layer comprising a hydrophilic polymeric matrix and a pharmaceutical composition comprising as the active agent at least one hydrophilic therapeutic or immunogenic peptide, polypeptide, or protein, the at least one drug reservoir layer is formulated in a dry form or as a hydrogel.

According to one embodiment in the system the electrode cartridge is adapted to generate a plurality of micro-channels of uniform shape and dimensions, or the electrical energy is of radio frequency.

According to a further embodiment the hydrophilic polymeric matrix is selected from the group consisting of hydrophilic biopolymers, hydrophilic synthetic polymers, derivatives and combinations thereof.

According to even another embodiment the hydrophilic biopolymer is selected from the group consisting of hydroxypropyl cellulose, carboxymethyl cellulose, hydroxyethyl cellulose, carrageenans, chitin, chitosan, alginates, collagens, gelatin, pectin, glycosaminoglycans (GAGs), proteoglycans, fibronectins, and laminins, preferably wherein the biopolymer is selected from the group consisting of collagens and carrageenans.

According to a further embodiment the hydrophilic synthetic polymer is selected from the group consisting of polyglycolic acid (PGA), polylactic acid (PLA), polypropylene oxide, polyethylene oxide, polyoxyethylene-polyoxypropylene copolymers, polyvinylalcohol, polyethylene glycol, and polyurethanes, preferably, wherein the hydrophilic synthetic polymer is polyethylene oxide.

According to one aspect of the present invention the active agent is selected from growth factors, hormones, cytokines, water-soluble drugs, antigens, antibodies, fragments and analogs thereof.

The active agent may also be selected from the group consisting of insulin, proinsulin, follicle stimulating hormone, insulin like growth factor-1, insulin like growth factor-2, platelet derived growth factor, epidermal growth factor, fibroblast growth factors, nerve growth factor, transforming growth factors, tumor necrosis factor, calcitonin, parathyroid hormone, growth hormone, bone morphogenic protein, erythropoietin, hemopoietic growth factors, luteinizing hormone, glucagon, clotting factors, anti- clotting factors, atrial natriuretic factor, lung surfactant, plasminogen activators, bombesin, thrombin, enkephalinase, relaxin A-chain, relaxin B-chain, prorelaxin, inhibin, activin, vascular endothelial growth factor, hormone receptors, growth factor receptors, integrins, protein A, protein D, rheumatoid factors, neurotrophic factors, CD proteins, osteoinductive factors, immunotoxins, interferons, colony stimulating factors, interleukins (ILs), superoxide dismutase, surface membrane proteins, T-cell receptors, decay accelerating factor, viral antigens, transport proteins, homing receptors, addressins, regulatory proteins, analogs, derivatives and fragments thereof, preferably wherein the active agent is growth hormone or insulin.

According to one embodiment the drug reservoir layer comprises a collagen and human growth hormone (hGH), or a collagen and human insulin, or polyethylene oxide and human growth hormone, or polyethylene oxide and human insulin, or a carrageenan and human growth hormone, or a carrageenan and human insulin.

According to a further embodiment the patch may further comprise at least one of the following layers: a backing layer, an adhesive, and a rate-controlling layer.

According to another embodiment the pharmaceutical composition may further comprise at least one component selected from the group consisting of protease inhibitors, stabilizers, anti-oxidants, buffering agents, and preservatives.

According to a further aspect of the present invention a system is used for sustained transdermal delivery of a hydrophilic therapeutic or immunogenic peptide, polypeptide or protein, the system comprises:
(i) an apparatus comprising:
   (a) an electrode cartridge comprising an array of electrodes; and
   (b) a main unit comprising a control unit, which is adapted to apply electrical energy to the electrodes when said electrodes are in vicinity of the skin, enabling ablation of stratum corneum in an area beneath the electrodes, thereby generating a plurality of micro-channels extending from the skin surface through all or a significant part of the stratum corneum; and
(ii) a patch adapted to be applied to the area of the skin where micro-channels are present after removal of the apparatus, the patch comprising at least one drug reservoir layer which comprises a hydrophilic polymeric matrix and a pharmaceutical composition comprising a hydrophilic therapeutic or immunogenic peptide, polypeptide, or protein, wherein the drug reservoir layer is formulated in a dry form or as a hydrogel.

According to an embodiment the hydrophilic polymeric matrix is selected from the group consisting of hydrophilic biopolymers, hydrophilic synthetic polymers, derivatives and combinations thereof.

According to another embodiment the hydrophilic biopolymer is selected from the group consisting of hydroxypropyl cellulose, carboxymethyl cellulose, hydroxyethyl cellulose, carrageenans, chitin, chitosan, alginates, collagens, gelatin, pectin, glycosaminoglycans (GAGs), proteoglycans, fibronectins, and laminins, preferably wherein the biopolymer is selected from the group consisting of collagens and carrageenans.

According to yet another embodiment the hydrophilic synthetic polymer is selected from the group consisting of polypropylene oxide, polyethylene oxide, polyoxyethylene-polyoxypropylene copolymers, polyvinylalcohol, polyurethanes, preferably wherein the hydrophilic synthetic polymer is polyethylene oxide.

According to a further embodiment the active agent is selected from the group consisting of growth factors, hormones, cytokines, water-soluble drugs, antigens, antibodies, fragments and analogs thereof.

According to yet another embodiment the active therapeutic or immunogenic agent is selected from the group consisting of insulin, proinsulin, follicle stimulating hormone, insulin like growth factor-1, insulin like growth factor-2, platelet derived growth factor, epidermal growth factor, fibroblast growth factors, nerve growth factor, transforming growth factors, tumor necrosis factor, calcitonin, parathyroid hormone, growth hormone, bone morphogenic protein, erythropoietin, hemopoietic growth factors, luteinizing hormone, glucagon, clotting factors, anti- clotting factors, atrial natriuretic factor, lung surfactant, plasminogen activators, bombesin, thrombin, enkephalinase, relaxin A-chain, relaxin B-chain, prorelaxin, inhibin, activin, vascular endothelial growth factor, hormone receptors, growth factor receptors, integrins, protein A, protein D, rheumatoid factors, neurotrophic factors, CD proteins, osteoinductive factors, immunotoxins, interferons, colony stimulating factors, interleukins (ILs), superoxide dismutase, T-cell receptors, surface membrane proteins, decay accelerating factor, viral antigens, transport proteins, homing receptors, addressins, regulatory proteins, analogs, derivatives and fragments thereof, preferably wherein the therapeutic agent is growth hormone or insulin.

According to a further aspect, the drug reservoir layer comprises a collagen and human growth hormone, or a collagen and human insulin, or polyethylene oxide and human growth hormone, or polyethylene oxide and human insulink, or carrageenan and human growth hormone, or carrageenan and human insulin.

According to another embodiment the patch further comprises at least one of the following layers: a backing layer, an adhesive, and a rate-controlling layer.

According to yet another embodiment the pharmaceutical composition further comprises at least one component selected from the group consisting of protease inhibitors, stabilizers, anti-oxidants, buffering agents and preservatives.

It should be appreciated that the patch according to the invention may be of any suitable geometry provided that it is adapted for stable and, optionally microbiologically controlled, aseptic or sterile, storage of the drug species prior to its use. The patch further comprises at least one of the following layers: a backing layer, an adhesive, and a rate-controlling layer.

Therapeutic blood concentrations of the active therapeutic or immunogenic agent may be maintained for at least 8 hours. Preferably, the therapeutic blood concentrations are maintained for at least 10 hours. As disclosed in the examples herein below, affixing a patch comprising a collagen film and hGH at a dose of 200 µg to a region of skin of rats or guinea pigs in which skin micro-channels were generated achieved hGH blood levels of 10-50 ng/ml for about 10 hours. It will be understood that similar hGH blood levels in guinea pigs were maintained for only approximately 5 hours when the same dose of hGH was transdermally administered using the apparatus of the invention in conjunction with a printed patch devoid of a polymeric matrix but comprising dry composition of hGH. The system of the present invention thus provides sustained and extended delivery of an active agent.

The present invention will be more fully understood from the following figures and detailed description of the preferred embodiments thereof.

### BRIEF DESCRIPTION OF THE FIGURES

FIG. **1** shows the permeation of human growth hormone (hGH; 70-90 µg) from a patch containing hGH-collagen film (A) or from a hGH printed patch (0) through porcine skin in which skin micro-channels were generated by ViaDerm and the permeation was detected by cumulative hGH amounts in an in-vitro assay.
FIG. **2** shows the permeation of hGH (70-90 µg) from a patch containing hGH-collagen film (▲) or from a hGH printed patch (◆) through porcine skin in which skin micro-channels were generated by ViaDerm and the permeation was detected by hGH transdermal flux in an in-vitro assay.
FIG. **3** shows the hGH blood levels after transdermal application of a printed patch containing 150 µg hGH (◆) or a patch containing 200 µg hGH-collagen film (□) on ViaDerm treated guinea pig skin.
FIG. **4** shows hGH blood levels after transdermal application of a patch containing 200 µg hGH-collagen film on ViaDerm treated guinea pig skin (□) or on ViaDerm treated rat skin (◆).
FIG. **5** shows blood glucose levels following subcutaneous administration of insulin (0) and transdermal application of a patch containing 0.4 IU insulin-collagen film on ViaDerm treated diabetic rat skin (■).
FIG. **6** shows blood glucose levels following application of 1.5 IU insulin-collagen patches on ViaDerm treated diabetic rat skin. Collagen A-Lispro (□); collagen A-NPH (0); collagen A-Ultra Lente (▲); and collagen B-Lispro (*).
FIG. **7** shows the permeation of human insulin from insulin-Vigilon® hydrogel patches through porcine skin in which skin micro-channels were generated by ViaDerm and the permeation was detected by cumulative insulin amounts in an in-vitro assay. 0.25 IU insulin in Vigilon® (◆); 0.5 IU insulin in Vigilon® (□); 2.5 IU insulin in Vigilon® (▲); and 5 IU insulin in Vigilon® (o).
FIG. **8** shows the transdermal flux of human insulin from insulin-Vigilon® hydrogel patches through porcine skin in which skin micro-channels were generated by ViaDerm and the permeation was detected by hGH transdermal flux in an in-vitro assay. 0.25 IU insulin in Vigilon® **(◆);** 0.5 IU insulin in Vigilon® (□); 2.5 IU insulin in Vigilon® (▲); and 5 IU insulin in Vigilon® (o).
FIG. **9** shows blood glucose levels after application of insulin-Vigilon® hydrogel patches to ViaDerm treated diabetic rat skin. Diabetic rats were injected subcutaneously with 0.1 IU of insulin and two hours later insulin-Vigilon® hydrogel patches were applied. Subcutaneous injection of insulin (●); Subcutaneous injection of insulin and 1.5 IU of insulin in Vigilon® (▲); Subcutaneous injection of insulin and 2.5 IU of insulin in Vigilon® (0).
FIG. **10** shows the amount of hGH released from a patch containing insulin-carrageenan film. The amount of hGH released was calculated as % of the initial amount of hGH added to carrageenan solution before film formation (initial). Carrageenan type I (◆); Carrageenan type II **(■).**
FIG. **11** shows the amount of insulin released from a patch containing insulin-carrageenan type II film. The amount of insulin released was calculated as % of the initial amount of insulin (5 IU) added to carrageenan solution before film formation.
FIG. **12** shows the permeation of insulin from a patch containing insulin-carrageenan film (▲) through porcine skin in which skin micro-channels were generated by ViaDerm and the permeation was detected by cumulative insulin amounts in an in-vitro assay. Insulin 1 IU (◆); Insulin 5 IU (■).

### DETAILED DESCRIPTION OF THE INVENTION

The present invention provides systems and methods for delivering hydrophilic active agents, particularly hydrophilic high molecular weight polypeptides or proteins through treated skin in which micro-channels have been generated.

It is now disclosed that use of a patch comprising a polymeric drug reservoir layer comprising a hydrophilic polymer or a protein and an active agent, when placed on an area of the skin pretreated by an apparatus that generates micro-channels, extends and improves the transdermal delivery of the active agent as compared to a medical patch comprising dried or lyophilized composition comprising the same active agent but devoid of the hydrophilic polymeric matrix. Moreover, the patch according to the principles of the present invention extends the delivery of the active agent if the delivery is compared to the delivery of the same dose of the same active agent when injected subcutaneously. The present invention, therefore, provides highly efficient systems and methods for sustained and slow delivery of hydrophilic high molecular weight proteins.

It is also disclosed that a patch comprising a hydrophilic polymer and a therapeutically active agent maintains the stability and activity of the active agent throughout the transdermal delivery, thus maintaining therapeutic blood concentrations for significantly extended periods of time and achieving extended therapeutic effect as compared to that obtained by subcutaneous injection.

The term "micro-channel" as used in the context of the present specification and claims refers to a hydrophilic pathway generally extending from the surface of the skin through all or a significant part of the stratum corneum and may reach into the epidermis or dermis, through which molecules can diffuse. It should be appreciated that after micro channels have been generated in the stratum corneum, the apparatus is removed from the skin, and the active agent is delivered from a patch subsequently placed on the skin into the systemic circulation.

The present invention incorporates devices and techniques for creating micro-channels by inducing ablation of the stratum corneum by electric current or spark generation, preferably at radio frequency (RF), including the apparatus referred to as ViaDerm or MicroDerm, as disclosed in one or more of the following: U.S. 6,148,232; US 5,983,135; US 6,597,946; US 6,611,706; US 6,708,060; and WO 2004/039428; the content of which is incorporated by reference as if set forth herein. It is however emphasized that although some preferred embodiments of the present invention relate to transdermal delivery obtained by ablating the skin by ViaDerm, substantially any method known in the art for generating channels in the skin of a subject may be used (see e.g. U.S. Pat. Nos. 5,885,211; 6,022,316; 6,142,939 and 6,173,202).

As the micro-channels are aqueous in nature, the system of the present invention is therefore highly suitable for delivery of hydrophilic macromolecules through the new skin environment, which is created by the ablation of the stratum corneum.

The terms "active agent" and "therapeutic or immunogenic agent" are used interchangeably herein to refer to a compound which, when administered to an organism (human or animal), induces a desired therapeutic effect by systemic action.

According to the invention, the system of the invention is suitable for transdermal delivery of peptides, polypeptides, and proteins.

A "peptide" refers to a polymer in which the monomers are amino acids linked together through amide bonds. "Peptides" are generally smaller than proteins, typically under 30-50 amino acids in total.

A "polypeptide" refers to a single polymer of amino acids, generally over 50 amino acids.

A "protein" as used herein refers to a polymer of amino acids typically over 50 amino acids. The peptides, polypeptides, or proteins that may be used as active agents in the present invention may be naturally occurring peptides, polypeptides, or proteins, modified naturally occurring peptides, polypeptides, or proteins, or chemically synthesized peptides, polypeptides, or proteins that may or may not be identical to naturally occurring peptides, polypeptides, or proteins. Derivatives, analogs and fragments of the peptides, polypeptides or proteins are encompassed in the present invention so long as they retain a therapeutic or immunogenic effect.

Disclosed is a system for facilitating transdermal delivery of an active agent through skin of a subject comprising: an apparatus capable of generating at least one micro-channel in an area on the skin of the subject, and a patch comprising at least one drug reservoir layer, the drug reservoir layer comprises a polymeric matrix and a pharmaceutical composition comprising as an active agent a therapeutic or immunogenic peptide, polypeptide, or a protein.

Suitable active agents for use in conjunction with the principles of the invention include therapeutic or immunogenic peptides, polypeptides, proteins, and water-soluble drugs including, but not limited to, insulin, proinsulin, follicle stimulating hormone, insulin like growth factor-1 and insulin like growth factor-2, platelet derived growth factor, epidermal growth factor, fibroblast growth factors, nerve growth factor, transforming growth factors, tumor necrosis factor, calcitonin, parathyroid hormone, growth hormone, bone morphogenic protein, erythropoietin, hemopoietic growth factors luteinizing hormone, glucagon, clotting factors such as factor VIIIC, factor IX, tissue factor, and von Willebrand factor, anti-clotting factors such as Protein C, atrial natriuretic factor, lung surfactant, a plasminogen activator such as urokinase or tissue-type plasminogen activator, bombesin, thrombin, enkephalinase, mullerian-inhibiting agent, relaxin A-chain, relaxin B-chain, prorelaxin, Dnase, inhibin, activin, vascular endothelial growth factor, receptors for hormones or growth factors, integrins, protein A or D, rheumatoid factors, a neurotrophic factor such as bone-derived neurotrophic factor (BDNF), neurotrophin-3, -4, -5, and -6 (NT-3, NT-4, NT-5, or NT-6), CD proteins such as CD-3, CD-4, CD-8, and CD-19, osteoinductive factors, immunotoxins, an interferon such as interferon-alpha, -beta, and -gamma, colony stimulating factors (CSFs), e.g., M-CSF, GM-CSF, and G-CSF, interleukins (ILs), e.g., IL-1 to IL-10, superoxide dismutase, T-cell receptors, surface membrane proteins, decay accelerating factor, viral antigens such as, for example, a portion of the AIDS envelope, transport proteins, homing receptors, addressins, regulatory proteins, antibodies, analogs, and fragments of any of the above-listed polypeptides.

The term "analog" as used herein refers to peptides, polypeptides or proteins comprising altered sequences by amino acid substitutions, additions, deletions, or chemical modifications of the naturally occurring peptides, polypeptides, or proteins. By using "amino acid substitutions", it is meant that functionally equivalent amino acid residues are substituted for residues within the sequence resulting in a silent change. For example, one or more amino acid residues within the sequence can be substituted by another amino acid of a similar polarity, which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such substitutions are known as conservative substitutions. Additionally, a non-conservative substitution may be made in an amino acid that does not contribute to the biological activity of the peptide, polypeptide or protein.

According to the invention, the patch comprises at least one drug reservoir layer, in which the active agent is imbedded or non-covalently bound. Various polymers may be used to form the drug reservoir layer and include biopolymers and hydrophilic synthetic polymers.

The biopolymers, which may be used according to the invention include, but are not limited to, polysaccharides, particularly cellulose derivatives such as, for example, hydroxypropyl cellulose, carboxymethyl cellulose, and hydroxyethyl cellulose, chitin and/or chitosan, alginates; collagens; gelatin; pectin; glycosaminoglycans (GAGs); proteoglycans; fibronectins; carrageenans; and laminins (see, for example, U.S. Pat. Nos. 5,418,222; 5,510,418; 5,512,301; 5,681,568; 6,596,293; 6,565,879 and references therein and Curr. Pharm. Biotechnol., 2003, 4(5): 283-302; Crit. Rev. Ther. Drug Carrier Syst., 2001, 18(5): 459-501; Eur. J. Pharm. Sci., 2001, 14(3): 201-7; Adv. Drug Deliv. Rev., 2001, 51 (1-3): 81-96; and Int. J. Pharm., 2001, 221(1-2): 1-22).

According to an exemplary embodiment disclosed herein below, the drug reservoir layer can be produced from a solution of soluble collagen. Soluble collagen is a collagen that has an average molecular weight of less than 400,000, preferably having a molecular weight of about 300,000. One of the preferred characteristics of the soluble collagen is that it possesses a minimal amount of cross-linking, i.e., 0.5% or less. A particularly suitable soluble collagen is Vitrogen® (Cohesion Technologies Inc., Palo Alto, CA). It will be understood that other form of collagen, namely atelopeptide form of collagen, may also be used in the present invention. Atelopeptide collagen is a collagen that is free of telopeptide, which is a peptide located at one end of purified collagen often associated with immunogenicity. A solution of the telopeptide form of collagen can be converted to the atelopeptide form of collagen via hydrolysis using organic acid.

Generally, the biopolymers have charged or highly polar groups which enable them to bind the active agents. The biopolymer may be chemically modified to change its binding affinity for a selected active agent so as to improve the binding affinity to the active agent. However, according to the principles of the present invention, the polymeric matrix does not necessarily bind the active agent. The active agent may be imbedded or non-covalently bound to the polymeric matrix to form the drug reservoir layer.

Hydrophilic synthetic polymers that may be used according to the invention include biodegradable and non-degradable polymers including, but not limited to, polyglycolic acid (PGA) polymers, polylactic acid (PLA) polymers, polypropylene oxide, polyethylene oxide, polyoxyethylene-polyoxypropylene copolymers, polyvinylalcohol, polyethylene glycol, polyurethanes, for example, polyurethanes based on diisocyanate/polyglycol and glycol linkages wherein the glycol is polyethylene glycol, and other hydrophilic synthetic polymers known in the art. It should be appreciated to one skilled in the art that chemical conjugates whereby biopolymers are conjugated with hydrophilic synthetic polymers to form the drug reservoir layer are also encompassed in the present invention. For example, U.S. Pat. No. 5,510,418 discloses biocompatible conjugates comprising chemically derivatized GAGs chemically conjugated to hydrophilic synthetic polymers. Thus, a conjugate comprising a GAG covalently bound to a hydrophilic synthetic polymer may be further bound to another biopolymer such as collagen to form a three component conjugate. The polymeric materials of the invention do not need to be cross-linked, though cross-linking is possible.

Typically, the number of drug reservoir layers is determined by the desired release characteristics. Generally, more layers produce more steady and more sustained release of the active agent. The concentration of the active agent in the different layers may be varied and the thickness of the different layers need not be the same. Additionally, the drug reservoir layer may comprise one or more active agents so as to achieve a desired therapeutic or immunogenic effect.

The principles of the invention are exemplified herein below by a dry formulation of the drug reservoir layer, i.e., a film containing collagen and the active agent. U.S. Pat. No. 5,418,222, which is incorporated by reference as if fully set forth herein, discloses single and multiple collagen films that are useful for controlled release of pharmaceuticals. However, it should be appreciated that the drug reservoir layer may also be formulated in a semi-dry form, in a liquid form or in a hydrogel form. Hydrogels are macromolecular networks that absorb water but do not dissolve in water. That is, hydrogels contain hydrophilic functional groups that provide water absorption, but the hydrogels are comprised of cross-linked polymers that give rise to aqueous insolubility. Generally, hydrogels are composed of hydrophilic, preferably cross-linked, polymers such as, for example, polyurethanes, polyvinyl alcohol, polyacrylic acid, dextran, cellulose, alginate, chitin, chitosan, agar, agarose, carrageenan, polyoxyethylene, polyvinylpyrrolidone, poly(hydroxyethyl methacrylate) (poly(HEMA)), copolymer or mixtures thereof. In addition, any other formulation, which maintains the polymer properties of stability and retention of the active agent, is also conceivable.

Typically, the drug reservoir layers such as those formulated in a film, are thin, flexible, and conformable to provide intimate contact with a body skin, are capable of hydration and also are able to release an active agent from the reservoir at rates sufficient to achieve therapeutically effective transdermal fluxes of the agent. As the apparatus of the invention creates hydrophilic micro-channels through which exudates are released, these exudates release the drug contained within the drug reservoir layer.

The patch may comprise one or more rate controlling layers, which are usually microporous membranes. Rate controlling layers comprise biopolymers and/or synthetic polymers. The rate controlling layers are devoid of an active agent. Representative materials useful for forming rate-controlling layers include, but are not limited to, polyolefins such as polyethylene and polypropylene, polyamides, polyesters, ethylene-ethacrylate copolymer, ethylene-vinyl acetate copolymer, ethylene-vinyl methylacetate copolymer, ethylene-vinyl ethylacetate copolymer, ethylene-vinyl propylacetate copolymer, polyisoprene, polyacrylonitrile, ethylene-propylene copolymer, cellulose acetate and cellulose nitrate, polytetrafluoroethylene ("Teflon"), polycarbonate, polyvinylidene difluoride (PVDF), polysulfones, and the like.

The various layers contact each other by any method known in the art. One such method is to place layers adjacent to each other and apply pressure to the outer sides of the layers to force the layers together. Another method is to coat the surface of each of the layers to be contacted with a solvent, such as water, before placing the layers together. In this way, a thin portion of each surface will become soluble thereby producing adhesion upon contact. Another method is to use a known adhesive on one or more of the contacting surfaces. Preferably, the adhesive is one that will not interfere with the delivery of the active agent from the drug reservoir layer.

According to the invention, a patch is used to administer the active agent, in which case the active agent is present in one or more drug reservoir layers. The drug reservoir layer may itself have adhesive properties, or may further comprise an adhesive layer attached to the drug reservoir layer. The patch may further comprise a backing layer.

Typically, a backing layer functions as the primary structural element of a transdermal system and provides flexibility and, preferably, occlusivity. The material used for the backing layer should be inert and incapable of absorbing an active agent or any component of a pharmaceutical composition contained within the drug reservoir layer. The backing layer preferably comprises a flexible elastomeric material that serves as a protective covering to prevent loss of the active agent via transmission through the upper surface of the patch, and will preferably impart a degree of occlusivity to the system, such that the area of the body surface covered by the patch becomes hydrated during use. The material used for the backing layer should permit the device to follow the contours of the skin and be worn comfortably on areas of skin such as at joints or other points of flexure, that are normally subjected to mechanical strain with little or no likelihood of the device disengaging from the skin due to differences in the flexibility or resiliency of the skin and the device. Examples of materials useful for the backing layer are polyesters, polyethylene, polypropylene, polyurethanes, polyether amides, and the like.

During storage and prior to use, the patch may include a release liner. Immediately prior to use, this layer is removed so that the patch may be affixed to the skin. The release liner should be made from a drug or active agent impermeable material, and is a disposable element, which serves only to protect the patch prior to application.

According to the principles of the invention, the pharmaceutical composition comprises a pharmaceutically acceptable carrier.

The term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic agent is administered. Carriers are more or less inert substances when added to a pharmaceutical composition to confer suitable consistency or form to the composition.

As used herein a "pharmaceutically acceptable carrier" may be aqueous or nonaqueous solutions, suspensions, or emulsions. Examples of aqueous carriers include water, saline and buffered media, alcoholic/aqueous solutions, emulsions or suspensions.

To optimize desirable characteristics of a pharmaceutical composition, various additives may be optionally included in the pharmaceutical composition. Thus, to improve the stability of the active agent, a suitable stabilizing agent can be added. Suitable stabilizing agents include, but are not limited to, most sugars, preferably mannitol, lactose, sucrose, trehalose, and glucose. In order to improve water absorption, hygroscopic additives may be added as well. To produce a pH that is compatible with a particular active agent being used, a suitable buffer can be used. Suitable buffers include most of the commonly known and utilized biological buffers, including acetate, phosphate or citrate buffer. A compatible pH is one that maintains the stability of an active agent, optimizes its therapeutic effect or protects against its degradation. A suitable pH is generally from about 3 to about 8, preferably from about 5 to about 8, and most preferably a suitable pH is about neutral pH of from about 7.0 to about 7.5. Additionally, protease inhibitors, anti-oxidants, and preservatives, alone or in combination, may be added as well.

The pharmaceutical composition comprising an active agent may be incorporated into the solution of the biopolymer or hydrophilic synthetic polymer during film formation, hydrogel formation, or any other formulation of the drug reservoir layer, or the pharmaceutical composition comprising an active agent may be added subsequently to the formation of the film, hydrogel, or other formulation of the polymeric matrix. In currently exemplary embodiments, hGH or human insulin solutions were each added to a collagen solution during collagen film formation. Generally, the drug solution or the drug/polymer solution is allowed to dry after film or hydrogel formation. The drying time is varied according to the temperature of drying. A suitable temperature is from about 15°C to 37°C.

The amount of a therapeutic or immunogenic agent necessary to provide the desired levels in serum can be determined by methods described herein below and by methods known in the art. Thus, the amount of a therapeutic or immunogenic agent in a pharmaceutically composition per patch can be varied in order to achieve a desired therapeutic effect.

### Devices and methods for enhancing transdermal delivery of an active agent

The system of the present invention comprises an apparatus for enhancing transdermal delivery of an active agent. According to a principle of the invention the apparatus is used to generate a new skin environment through which an active agent is delivered efficiently.

The term "new skin environment" as used herein, denotes a skin region created by the ablation of the stratum corneum and formation of at least one micro-channel, using the apparatus of the present invention.

U.S. Patent 6,148,232 to Avrahami, discloses an apparatus for applying electrodes at respective points on skin of a subject and applying electrical energy between two or more of the electrodes to cause resistive heating and subsequent ablation of the stratum corneum primarily in an area intermediate the respective points. Various techniques for limiting ablation to the stratum corneum are described, including spacing of the electrodes and monitoring the electrical resistance of skin between adjacent electrodes. The Device for Transdermal Drug Delivery and Analyte Extraction of the type disclosed in US 6,148,232, and various modifications to that invention including those disclosed in U.S. Pat. No. 5,983,135, 6,597,946, 6,611,706, 6,708,060, are mentioned.

According to some embodiments, the apparatus for enhancing transdermal delivery of a therapeutic or immunogenic agent comprises: an electrode cartridge, optionally removable, comprising a plurality of electrodes, and a main unit wherein the main unit loaded with the electrode cartridge is also denoted herein ViaDerm.

The control unit is adapted to apply electrical energy to the electrode typically by generating current flow or one or more sparks when the electrode cartridge is in vicinity of the skin. The electrical energy in each electrode within the electrode array causes ablation of stratum corneum in an area beneath the electrode, thereby generating at least one micro-channel. Preferably, the electrical energy is of Radio frequency (RF).

The control unit comprises circuitry which enables to control the magnitude, frequency, and/or duration of the electrical energy delivered to an electrode, in order to control current flow or spark generation, and consequently to control the dimensions and shape of the resulting micro-channel. Typically, the electrode cartridge is discarded after one use, and as such is designed for easy attachment to the main unit and subsequent detachment from the unit.

To minimize the chance of contamination of the cartridge and its associated electrodes, attachment and detachment of the cartridge is performed without the user physically touching the cartridge. Preferably, cartridges are sealed in a sterile cartridge holder, which is opened immediately prior to use, whereupon the main unit is brought in contact with a top surface of the cartridge, so as to engage a mechanism that locks the cartridge to the main unit. A simple means of unlocking and ejecting the cartridge, which does not require the user to touch the cartridge, is also provided.

Optionally the electrode cartridge may further comprise means to mark the region of the skin where micro-channels have been created, such that a patch can be precisely placed over the treated region of the skin. It is noted that micro-channel generation (when practiced in accordance with the techniques described in the above-cited US patents to Avrahami et al. and patent applications, assigned to the assignee of the present patent application) does not generally leave any visible mark, because even the large number of micro-channels typically generated are not associated with appreciable irritation to the new skin environment.

According to some embodiments of the present invention, for other applications the micro-channels may be generated separately or simultaneously with the application of a patch. Among the other applications, the system may include a patch comprising an adhesive cut-out template which is placed on the skin, and through which the cartridge is placed to treat the region of skin exposed through the template. The active agent, contained within a patch according to embodiments of the present invention, is attached to the template, which is to be placed over the treated region of skin. In these applications, after removing a protective backing layer, the template portion of the patch is placed on the skin and secured by the adhesive. An electrode cartridge is then affixed to the handle, the user holds the handle so as to place the cartridge against the region of skin inside the template, and the electrodes are energized to treat the skin. Subsequently, the cartridge is discarded. A protective covering is then removed from the patch by pulling on a tab projecting from the covering, so as to concurrently lift and place the patch over the treated region of skin. It is noted that the integration of the template and the patch into a single unit assists the user in accurately placing the patch onto the treated area of skin. Utilizing the system of the invention in this manner becomes advantageous for disinfected applications.

For still other applications, an integrated electrode/medicated pad cartridge is used to provide a practical apparatus as disclosed in U.S. Pat. No. 6,611,706, which is assigned to the applicant of the present patent invention . In these applications, the cartridge comprises an electrode array, a controlled unit and a medicated pad. Accordingly, no template is typically required. The user places the electrodes against the skin and this contact is sufficient to initiate current flow or spark formation within the electrode and the subsequent formation of micro-channels. An adhesive strip, coupled to the bottom of the medicated pad, comes in contact with and sticks to the skin when the electrodes are placed against the skin. A top cover on the medicated matrix is coupled to the electrode region of the cartridge, such that as the electrode region, fixed to the handle, is removed from the skin the top cover is pulled off the medicated pad and the pad is concurrently folded over the treated region of skin. This type of application eliminates the need for the user to touch any parts of the electrode cartridge or the medicated pad, thus substantially reducing or eliminating the likelihood of the user contaminating the apparatus.

A current may be applied to the skin in order to ablate the stratum corneum by heating the cells. Spark generation, cessation of spark generation, or a specific current level may be used as a form of feedback, which indicates that the desired depth has been reached and current application should be terminated. For these applications, the electrodes are preferably shaped and/or supported in a cartridge that is conducive to facilitating ablation of the stratum corneum and the epidermis to the desired depth, but not beyond that depth. Alternatively, the current may be configured so as to ablate the stratum corneum without the generation of sparks.

Generally preferred embodiments of the present invention typically make use of apparatus described in U.S. Pat. No. 6,611,706 entitled "Monopolar and bipolar current application for transdermal drug delivery and analyte extraction," which is assigned to the applicant of the present invention. For example, U.S. Pat. No. 6,611,706 describes maintaining the ablating electrodes either in contact with the skin, or up to a distance of about 500 microns therefrom. Thus, the term "in vicinity" of the skin as used throughout the specification and claims encompasses a distance of 0 to about 500 microns from the electrodes to the skin surface. The application further describes spark-induced ablation of the stratum corneum by applying a field having a frequency between about 10 kHz and 4000 kHz, preferably between about 10 kHz and 500 kHz.

Alternatively or additionally, preferred embodiments of the present invention Use apparatus described in the U.S. Pat. No. 6,708,060 entitled "Handheld apparatus and method for transdermal drug delivery and analyte extraction,"

The cartridge supports an array of electrodes, preferably closely-spaced electrodes, which act together to produce a high micro-channel density in an area of the skin under the cartridge. Typically, however, the overall area of micro-channels generated in the stratum corneum is small compared to the total area covered by the electrode array.

A concentric electrode set is formed by employing the skin contact surface of the cartridge as a return path for the current passing from the electrode array to the skin. Preferably, the cartridge has a relatively large contact surface area with the skin, resulting in relatively low current densities in the skin near the cartridge, and thus no significant heating or substantial damage to the skin at the contact surface.

In proximity to each electrode in the electrode array, by contrast, the high-energy applied field typically induces very rapid heating and ablation of the stratum corneum.

Also disclosed is a method for sustained delivery of an active agent using a transdermal delivery system according to the principles of the invention. Typically, the procedure for forming new skin environment comprises the step of placing over the skin the apparatus for generating at least one micro-channel. Preferably, prior to generating the micro-channels, the treatment sites will be swabbed with sterile alcohol pads. More preferably, the site should be allowed to dry before treatment.

According to certain examples the type of apparatus used to generate micro-channels is disclosed in U.S. Pat. Nos. 6,148,232 and 6,708,060. The apparatus containing the electrode array is placed over the site of treatment, the array is energized by RF energy, and treatment is initiated. In principle, the ablation and generation of micro-channels is completed within seconds. The apparatus is removed after micro-channels are generated at limited depth, preferably limited to the depth of the stratum corneum and the epidermis. A patch according to the principles of the present invention is attached to the new skin environment.

Also disclosed is a method for sustained transdermal delivery of a therapeutic or immunogenic agent, the method comprising:
(i) generating at least one micro-channel in a region of the skin of a subject;
(ii) affixing a patch to the region of the skin in which the at least one micro-channel is present, the patch comprises at least one drug reservoir layer, wherein the drug reservoir layer comprises a polymeric matrix and a therapeutic or immunogenic peptide, polypeptide, or protein;
(iii) and achieving a therapeutically effective blood concentration of the peptide, polypeptide, or protein for at least 6 hours.

As defined herein "therapeutically effective blood concentration" means a concentration of an active therapeutic or immunogenic agent, which results in a therapeutic effect.

The term "therapeutic" is meant to include amelioration of the clinical condition of a subject and/or the protection, in whole or in part, against a pathological condition or disease. As the present invention encompasses immunogenic agents as active ingredients, the term therapeutic used throughout the specification includes the induction of an immune response such as, for example, cellular immune response and/or humoral immune response.

According to one example, the active agent is hGH. As disclosed herein below, blood concentrations of hGH in the range of 10 ng/ml to 50 ng/ml in rats and in guinea pigs were obtained within approximately 2-4 hours for a period of about 10 hours when 200 µg hGH were administered in a collagen film. In contrast, when 200 µg hGH were administered to guinea pigs in a printed patch devoid of a collagen film, similar hGH blood concentrations were obtained within 1 hour for only 5 hours (see Example 4 herein below). Thus, the method of transdermal delivery according to the principles of the present invention provides sustained delivery of hGH.

In another exemplary embodiment, the active agent is human insulin. According to the invention, human insulin (0.4 IU) transdermally administered to diabetic rats by the system of the invention normalized blood glucose levels 2.5 hours after patch application, and such normal levels were maintained for about 9 hours (see Example 7). In contrast, subcutaneous administration of insulin at the same dose reduced blood glucose levels in diabetic rats 1 hour after injection, and the normal glucose levels were maintained for 5 hours. Thus, transdermal delivery according to the present invention provides sustained delivery of insulin culminating in a therapeutic effect.

The present invention thus encompasses patches comprising a therapeutic or immunogenic peptide, polypeptide or protein such as, for example, hGH or human insulin, which is impregnated or embedded within a polymer, preferably biopolymer or hydrophilic polymer or a combination thereof. Use of such patches in conjunction with the apparatus of the present invention results in achieving therapeutic blood concentrations for at least 6 hours, preferably for at least 8 hours, and more preferably for at least 10 hours.

Additionally, a therapeutic blood concentration of a therapeutic peptide, polypeptide or protein is determined by the clinical state of a subject. For example, a therapeutic blood concentration of insulin is determined by the blood glucose level of a diabetic subject. Also, a therapeutic blood concentration of hGH is determined by the growth rate in children or by the blood level of IGF-1 in adults. Thus, based on the clinical state of a subject, a clinician would determine a therapeutic concentration of an active agent as known in the art. Similarly, the duration of treatment or duration of exposure to the therapeutic agent will be determined by the clinician taking into consideration the disease to be treated, as well as secondary factors including the gender age, and general physical condition of the patient.

Having now generally described the invention, the same will be more readily understood through reference to the following examples, which are provided by way of illustration and are not intended to be limiting of the present invention.

### EXAMPLE 1

### Preparation of hGH-collagen film based patches

The collagen used herein was Vitrogen^{®} 100 (3mg/ml, Cohesion Technologies Inc, Palo Alto, CA, USA). Human growth hormone was Genotropin^{®} (5.3mg/16 IU, Pharmacia and Upjohn, Stockholm, Sweden). Phosphate Buffered Saline (PBS) was obtained from Biological Industries (Kibbutz Beit Haemak, Israel).

Some of the collagen patches consisted of the following: Backing liner BLF 2080 3mil (Dow film) coated with adhesive (National-Starch Duro-Tak 387-251; Zutphen, The Netherlands), covered with perforated SIL-K silicon strip 25 mil (Dgania Silicone, Dgania, Israel), opening side of 1.4 cm², and Release liner Rexam 78CD (Rexam Inc., Bedford Park, IL, USA).

Collagen (Vitrogen®) solution was gently mixed with PBSx10 and NaOH 0.1M at a volume ratio of 8:1:1 for collagen: PBSx10: NaOH, respectively. The desired amount of hGH was added from a stock solution of 14 mg/ml. The collagen-hGH solution at a final volume of 300-400 µl was poured to the patch template and placed at 37°C for 30 minutes until gelation occurred. The patches were then air dried, covered with Parafilm, packed with laminate bag, silica gel and argon. The patches were kept at 4°C until used.

### EXAMPLE 2

### Recovery of hGH from the collagen film based patches

Human GH content in collagen films was determined by extraction of the hGH to a PBS solution and by quantitative analysis using size exclusion HPLC (Phenomenex SEC, 250x4.6mm, Phenomenex). The recovery of hGH from the collagen film is summarized in Table 1.

**Table 1. Recovery of hGH from collagen based patches.**

| **Drug** | **Base of film** | **Recovery of drug (% of initial amount)** |
|---|---|---|
| hGH | Backing liner (Dow film) | 94 ± 7 |
| hGH | Adhesive (Duro-Tak) | 100 ± 21 |

Three hundred micrograms of hGH were loaded on each patch. The recovery results are the mean±SD of three experiments. As shown in Table 1, over 90% of the initial amount was extracted from the films within 5 minutes. There was no significant difference in the amount extracted when the collagen was poured directly to the adhesive or to a backing liner, although the variation within the adhesive group was higher.

For studying the release kinetics, the collagen films were suspended in PBS (2 ml) and incubated at room temperature. At different time points, the buffer was replaced with a fresh buffer and the amount of hGH was analyzed by size exclusion HPLC.

Collagen of the Vitrogen® type released the majority of the hGH within 1 hr. The release of hGH from collagen-hGH patches through ViaDerm treated skin was next examined.

### EXAMPLE 3

### In vitro permeation study of hGH through skin

The permeability of hGH through porcine skin was measured in vitro with a Franz diffusion cell system (house made). The diffusion area was 2 cm². Dermatomized (300-500 µm, Electric Dermatom, Padgett Instruments Ltd, Kansas, MI, USA) porcine skin was excised from slaughtered white pigs (breeding of Landres and Large White, locally grown in Kibbutz Lahav, Israel). Transepidermal water loss measurements (TEWL, Dermalab Cortex Technology, Hadsund, Denmark) were performed and only those pieces with TEWL levels less than 15 g/m² /h were mounted in the diffusion cells.

For the preparation of a printed patch with a required amount of hGH, the volume of each droplet was calculated according to the concentration of the hGH in the solution and accordingly the syringe's plunger displacement, which is required per one droplet printing, was adjusted, wherein the range of 0.035 - 0.105 mm corresponded to 0.09 - 0.18 µl. This range of displacement was fed into a Basic program that controlled the printing. Next, the Backing layer film (DOW BLF2080™, The Dow Chemical Company, MI, USA) was placed flat with the bright side up on a flat metal block. The syringe containing hGH solution was loaded into the XYZ dosing machine, which then placed measured hGH drops on the backing liner. It should be noted that within a few minutes the drops started to dry consecutively. Once the 144 dots array of printed droplets was formed the printing of new array started on a new position. According to this procedure it was possible to form up to 6 arrays on a 5.5 x 1.6 inches backing liner. Sections, 2x2 cm², of the printed 144 dotted arrays were kept at 4°C in close vials.

Skin micro channeling was performed using the ViaDerm™ instrument (U.S. Patent No. 6,148,232, WO 2004/039428 and references therein, the content of which is incorporated by reference as if fully set forth herein, Sintov et al. J. Cont. Release 89, 311-320, 2003). The density of the microelectrode array used in all the studies was 100 microelectrodes/cm². The device was applied twice on each location, so the density of the micro channels was 200/cm². The skin was treated with an applied voltage of 330V, frequency of 100kHz, two bursts, 700 microsec burst length, and no current limitation. Following ViaDerm application, the TEWL was measured again to control the operation.

The skin pieces were washed 3 times with PBS, and hGH printed patches or hGH collagen films were placed on the stratum corneum side. The skins plus the patches or films were then placed on the acceptor cell with the stratum corneum facing upwards, and the donor chambers were clamped in place. The acceptor cells were filled with phosphate buffered saline (PBS, pH 7.4) that contained 0.1% sodium azide (Merk, Dermstadt, Germany), 1% bovine serum albumin (Biological Industries, Kibutz Beit-Hamek, Israel) and protease inhibitors cocktail (1 tablet per 50 ml PBS, Complete Mini, Roch). Samples from the receiver solutions were collected into tubes at predetermined times for up to 20 hr period. The samples were kept at 4°C until analyzed. hGH analysis was performed by ELIZA kit (DSL-10-1900, Diagnostic Systems Laboratories, Inc. Webster, Texas, USA).

### Results

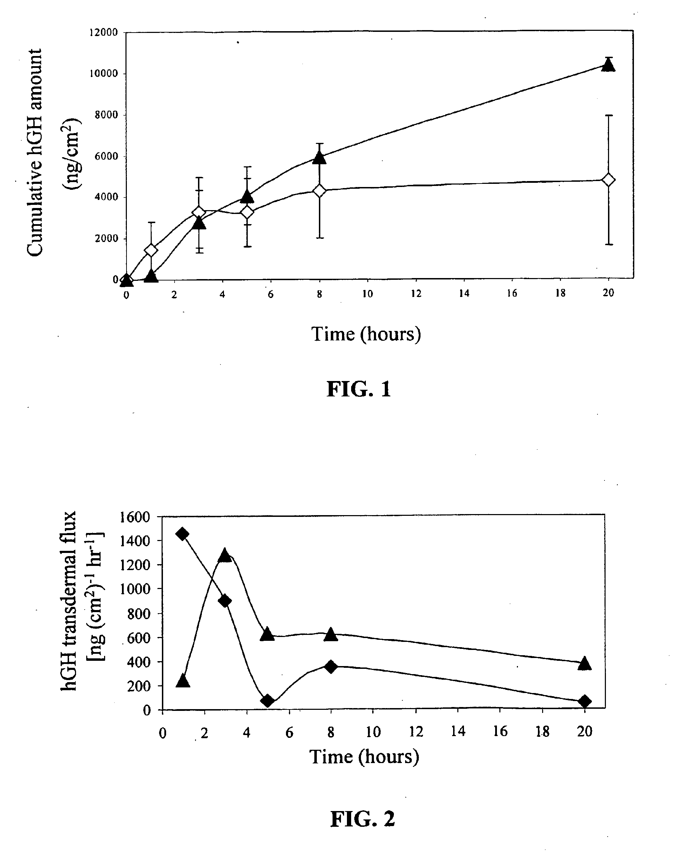

The cumulative permeability of hGH from printed patches or from collagen films through ViaDerm treated skin is shown in FIG. 1. As shown in FIG. 1, administration of hGH using a hGH-collagen film based patch resulted in a lower hGH permeation compared to that obtained with a hGH-printed patch one hour after film or printed patch administration. These results indicate that a delayed delivery was achieved when a hGH-collagen film based patch was used. However, at later periods of time administration of hGH using a hGH-collagen film based patch resulted in an increase of the cumulative hGH amount as a function of time, and the total permeation amount 20 hrs after hGH administration was two fold higher with the hGH-collagen film patch than with the hGH printed patch (FIG. **1**). The hGH transdermal flux values (amount permeated per cm² per hr) are shown in FIG. **2**. After 1 hr of hGH application, the flux of hGH from a printed patch was 6 times higher than that obtained with a collagen film. However, at time points from 3 hr to 20 hr, the flux of hGH from the collagen film was higher than that observed from the printed patch. The in-vitro results suggest that a sustained delivery of hGH can be attained with collagen films.

### EXAMPLE 4

### In vivo hGH Transdermal Delivery

Male Guinea pigs (500-800 grams, Dunkin Hartley, Harlan laboratories Ltd., Israel) and Male rats (350-400, Sprague Dawley, Harlan laboratories Ltd., Israel) were pre-medicated with IP injections of 10% ketamin/2% xylazine solution at a ratio of 70:30, 1 ml/kg. Anesthesia was maintained with either isofluorane or halothane gas. The abdominal skin hair was shaved carefully, and was cleaned with isopropyl alcohol. After 30 min, transepidermal water loss measurements (TEWL, Dermalab Cortex Technology, Hadsund, Denmark) were performed to check skin integrity. Skin micro channeling was performed by the use of the ViaDerm instrument with the conditions described in Example 3 herein above. TEWL was then measured again to control the operation. The treated skin was covered with either hGH printed patches or hGH-collagen film based patches and blood samples were withdrawn at 0, 2, 4, 6, 9, 12, and 15 hr post application from a preinserted carotid cannula in the guinea pig or from the rat tail.

### Results

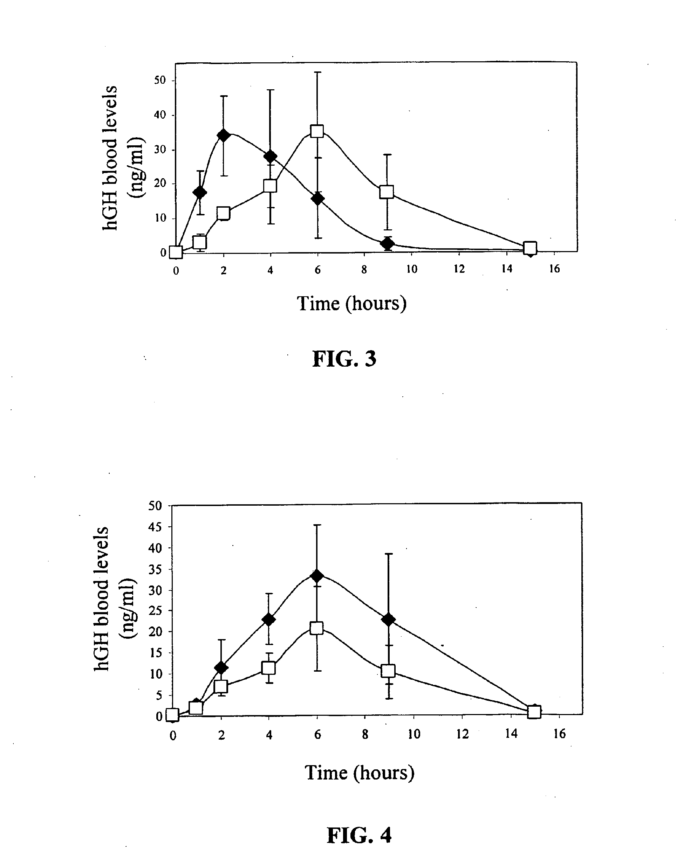

Delivery of hGH through ViaDerm treated guinea pig skin is shown in FIG. **3**. Embedding hGH in collagen resulted in sustained delivery as compared to a printed patch. The in-vivo findings correlate with the in-vitro results. Maximal delivery was obtained after 6 hrs with a collagen film and after 2 hrs with a printed patch (FIG. **3**). Moreover, hGH was not detected in the blood 9 hr after administration of the printed patch while significant levels were detected at that time after administration of the collagen based patches (FIG. **3**).

Delivery of hGH through ViaDerm treated guinea pig skin or through rat skin are shown in FIG. **4**. As shown in FIG. **4**, an extended delivery of hGH was observed also in rats. The curve profile in rat was similar to that of guinea pigs.

An extended delivery was also achieved when higher amounts (300 µg) of hGH were administered (see Table 2).

**Table 2. hGH serum levels after transdermal application to ViaDerm treated guinea pig**

| | hGH ng/ml | |
|---|---|---|
| Time | Collagen patch | Printed patch |
| 6 | 33.08 ± 10.90 | 42.55 ± 9.65 |
| 9 | 17.71 ± 6.78 | 4.90 ± 5.07 |
| 12 | 10* | 0* |
| 15 | 1.83 ± 1.60 | 0.22 ± 0.08 |

As shown in Table 2, similar hGH serum levels were observed 6 hrs after ViaDerm and patch application to guinea pigs (33.1±10.9 and 42.6±9.7 ng/ml for collagen film based patch and printed patch, respectively, Table 2). However, much higher hGH serum levels were observed in the collagen group at later time points (17.7±6.8 versus 4.9±5.1 ng/ml for collagen film based patch and printed patch, respectively, Table 2). Thus, the in-vivo results indicate that an extended release profile can be achieved by the use of biocompatible collagen films.

### EXAMPLE 5

### Preparation of insulin-collagen film based patches

For the preparation of insulin-collagen film based patches, the collagen used was either Vitrogen® 100 (3mg/ml, Cohesion Technologies Inc, Palo Alto, CA, USA) or Atelocollagen (6.5%, Koken Co, LTD, Tokyo, Japan). Human recombinant insulin Humolog^{®} (Lispro-100 IU/ml), Humulin N^{®} (NPH-100 IU/ml), and Humulin U^{®} (Ultra Lente (UL)-100 IU/ml) were purchased from Lilly (Lilly France S.A., Fegershein, France).

Phosphate Buffered Saline (PBS) was obtained from Biological Industries (Kibbutz Beit Haemak, Israel).

The template of the collagen patches consisted of a backing liner (BLF 2080 3mil, Dow film) at dimensions of 2.25cm².

Vitrogen® (collagen A) solution was gently mixed with PBSx10 and NaOH 0.1M at a volume ratio of 8:1:1 for collagen: PBSx10: NaOH, respectively. The desired amount of insulin was added from a stock solution of 100 IU/ml. The collagen insulin solution at a final volume of 300-320 µl was poured to the patch template and placed at 37°C for 60 minutes until gelation occurred. The patches were then air dried and packed with laminate bag, silica gel and argon. The patches were kept at 4°C until used.

Atelocollagen (collagen B) was diluted at 4°C to a desired concentration with PBS containing insulin. The amount of insulin was calculated according to the final selected amount in the patch. Insulin was diluted from a stock solution of 100 IU/ml.

The atelocollagen-insulin solution at a final volume of 300 µl was poured to the patch template and air-dried. The patches were packed and kept as described above.

### EXAMPLE 6

### Insulin content in insulin-collagen patches before and after transdermal application

ViaDerm Engineering Prototype was used. The diameter of the microelectrode array used in all the studies was 80 µm, and the density was 75 microelectrodes/cm². Rat skin was treated with an applied voltage of 330V, frequency of 100kHz, two bursts, 700 microsecond burst length, and no current limitation.

Insulin content in all the collagen patches was determined by extraction of the insulin to a PBS or HCl 0.1N solutions (for insulin-lispro or insulin-NPH/UL, respectively) and quantitated by reverse phase HPLC analysis.

### Results

Summary of the analysis of the insulin content in the collagen patches before and after transdermal application is shown in Table 3.

**Table 3. Insulin content within various insulin-collagen patches before and after transdermal delivery.**

| **Group** | **Input IU of insulin** | **IU in patch** | **Residual insulin in patch following transdermal application** |
|---|---|---|---|
| **Vitrogen 0.3% - Lispro** | 1.8 | 1.5 ± 0.1 | 0.6 ± 0.1 |
| **Atelocollagen 0.9% - Lispro** | 1.8 | 1.4 ± 0.1 | 0.3 ± 0.1 |
| **Vitrogen 0.3% - NPH** | 1.8 | 1.6 ± 0.1 | 0.6 ± 0.0 |
| **Vitrogen 0.3% - UL** | 1.8 | 1.3 ± 0.0 | 0.6 ± 0.0 |
| **Vitrogen 0.3% - Lispro, LD** | 0.6 | 0.4 ± 0.0 | 0.0 ± 0.0 |

As shown in Table 3, high dose patches (1.8 IU of insulin input) contained 1.5 IU insulin in average, which constitute 83% of the initial amount applied. Low dose (LD) patches (0.6 IU of insulin input) contained 0.4 IU insulin, which constitute 67% of initial amount applied. The 20-30 % of the initial insulin input that were not extracted were probably bound to collagen or to atelocollagen.

It should be noted that the discrepancy in insulin content within the different collagen patches was relatively small (standard deviation of 0-7%; Table 3). The residual insulin left within a patch following insulin transdermal application correlated with the amount of insulin in the collagen patch and with the duration of application of the patch placed on the skin. The 0.4 IU patches were placed on the skin for 12.5 hr and no residual insulin was found (Table 3). The 1.5 IU patches contained almost 4 times more insulin than the LD patches and were placed on the skin for a shorter period of time (8-10 hr). The residual insulin within these patches was found to be about 20% and 40% of the insulin input amount for atelocollagen patches and Vitrogen® patches, respectively (Table 3).

These results indicate that insulin can be transdermally delivered from LD insulin-collagen patches at a maximal efficacy (100 % of the insulin input). Such delivery is somewhat lower when insulin is delivered from insulin-collagen patches, in which the insulin content is higher (1.5 IU insulin). However, higher efficacy may be obtained if one prolongs the duration of the patch application.

### EXAMPLE 7

### Bioactivity of insulin embedded in collagen films in diabetic rats

Male rats (300-325, Sprague Dawley, Harlan laboratories Ltd., Israel) were deprived of food and received water ad libitum 48 hr prior to patch applications. Streptozotocin (55 mg/kg in citric buffer, 0.1M, pH 4.5; Sigma, St. Louis, MO, USA) was injected IP to the rats 24 hr prior to patch application in order to induce diabetes.

Keto-Diastix-Glucose and Ketones urinalysis sticks, Glucometer^{®}, and blood glucose test strips were used (Ascensia Elite, Bayer).

The rats were defined as diabetic when 24 hr following the injections the glucose levels were above 300 mg/dl, and positive urine glucose and negative urine ketones were observed. The diabetic rats were IP injected with a 10% ketamin/ 2% xylazine solution at a ratio of 70:30, 1 ml/kg. Anesthesia was maintained with either isofluorane or halothane gas. The abdominal skin hair was shaved carefully, and was cleaned with isopropyl alcohol. After 30 min, transepidermal water loss measurements (TEWL, Dermalab Cortex Technology, Hadsund, Denmark) were performed to check skin integrity. Skin micro channeling was performed by the use of the ViaDerm instrument with the conditions described in Example 6. TEWL was then measured again to control the operation. The treated skin was covered with various insulin-collagen patches. Subcutaneous (SC) injections of 0.4 IU served as a positive control. Blood samples were obtained from the tip of the rat's tail and the level of blood glucose was determined at predetermined time points post application.

### Results

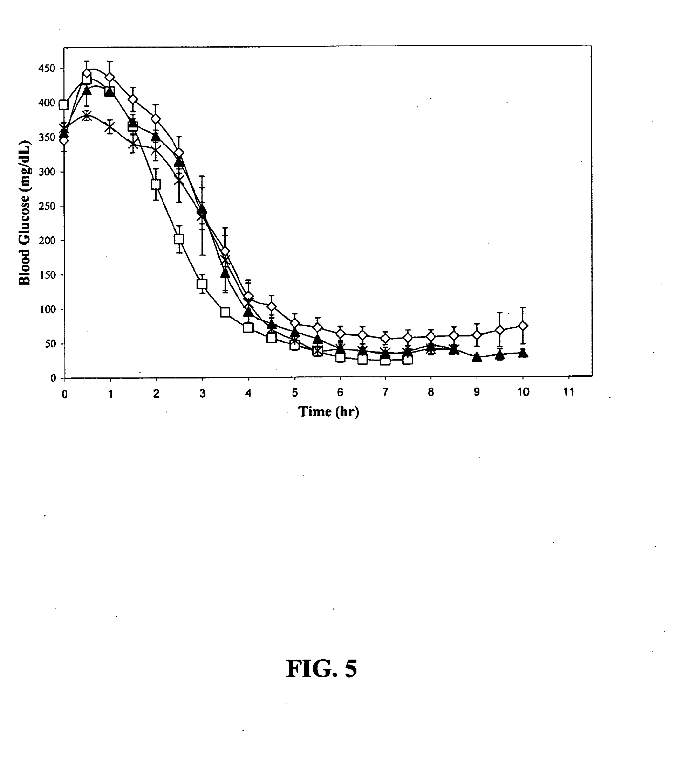

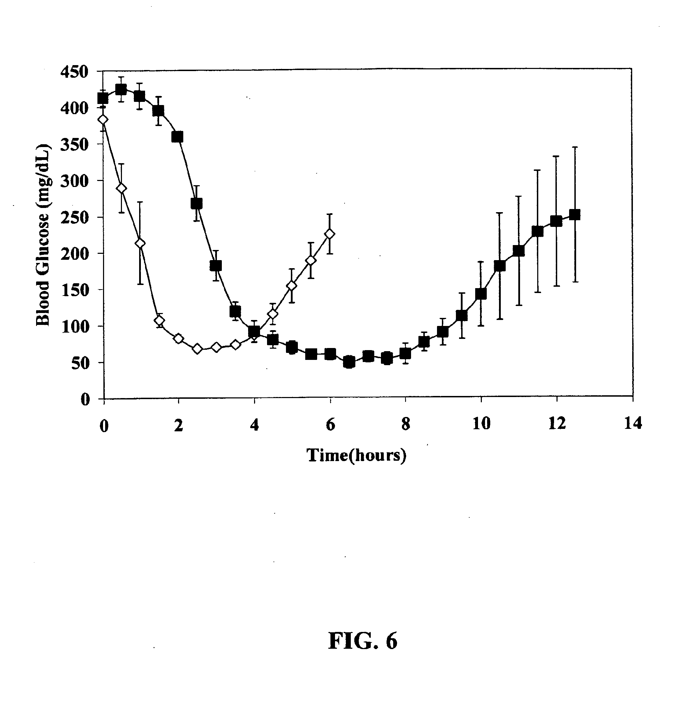

Blood glucose levels after application of insulin-collagen patches to micro-channeled skin of diabetic rats are shown in FIG. **5** and FIG. **6****.** As shown in FIG. **5****,** subcutaneous (SC) administration of 0.4 IU insulin to rats resulted in a rapid decrease in blood glucose levels, i.e., one hr after SC administration, glucose level was lower than 200 mg/dL. The SC effect continued for about 4 hrs, and the glucose levels were raised above 200 mg/dl afterwards (FIG. **5**). It should be noted that the normal glucose levels in rats are in the range of 100-200 mg/dl. In contrast, a delay in the decrease of blood glucose level was observed when insulin-collagen patches were applied as compared to SC administration of insulin. This delay in the decrease of blood glucose level was due to the re-hydration of the collagen patches and the diffusion of insulin through the micro channels generated. In addition, transdermal application of 0.4 IU insulin in collagen patches caused a sustained release of insulin, which was reflected by a sustained decrease (lower than 200 mg/dL) in blood glucose level; such low glucose levels continued for about 8 hrs (FIG. **5**).

Administration of 1.5 IU of insulin in insulin-collagen patches to rats resulted in a decrease of more than 90% of the initial glucose level (FIG. **6**) and in a hypoglycemic shock. As a result, the experiment was not continued for more than 10 hours (FIG. **6**). Thus, the optimal dose of insulin patches for studies in rats should be lower than 1.5 IU.

FIG. **6** shows the effect of various insulin-collagen patches placed on ViaDerm treated skin on blood glucose levels in diabetic rats. As shown in FIG. **6****,** two and a half hours after collagen A-lispro patch application, the levels of glucose decreased from about 400 mg/dl to about 200 mg/dl. The same decrease was achieved an hour later after collagen A-NPH or collagen A-Ultra Lente (UL) patch applications. This difference between collagen A-NPH or collagen A-Ultra Lente patches and collagen A-lispro patch was probably due to the different time period required for insulin to depart from the NPH or UL insulin-complex formulations. The application of collagen B-lispro resulted in blood glucose profile similar to collagen A-NPH or collagen A-UL patches. These results indicate that a delay in insulin effect can be obtained by the use of differently complexed insulin.

The present findings indicate that various insulin-collagen patches exhibit a delayed and extended delivery of insulin as reflected by delayed and extended blood glucose profile. As a result, the biological effect of insulin was longer than that obtained after SC injection.

### EXAMPLE 8

### Preparation of insulin-Vigilon® based patches

Vigilon^{®} hydrogel (C.R. BARD, Covington, GA, USA) was used for the preparation of the insulin patches. The human recombinant insulin Humulin^{®} R (Regular-100 IU/ml), was purchased from Lilly (Lilly France S.A., Fegershein, France). Phosphate Buffered Saline (PBS) was obtained from Biological Industries (Kibbutz Beit Haemak, Israel).

Squares of Vigilon^{®} hydrogel sheet were cut at dimensions of 2.25cm² and were prehydrated with insulin solution prior to the transdermal application. It was found that incubation of a Vigilon^{®} square with an insulin solution for 1 hr resulted in absorption of 0.2 ml of the insulin solution. Therefore, the Vigilon^{®} squares were incubated with insulin solutions that contained the desired final loading dose in 0.2 ml. The incubation solution volume for each patch was 2 ml. A stock solution of 100 IU/ml was diluted to the desired concentration with PBS. Thus, for example, in order to prepare 2.5 IU loaded patch, the Vigilon^{®} template was incubated for 1 hr with an insulin solution at a concentration of 12.5 IU/ml.

### EXAMPLE 9

### Content of insulin in insulin-Vigilon® patches

Insulin content in all the Vigilon® patches was determined by extraction of the insulin with PBS solutions and quantitative analysis by reverse phase HPLC (LUNA 5um C18, 150x4 mm, Phenomenex).

Summary of the analysis of the content of insulin in the Vigilon® patches is shown in Table 4.

**Table 4. Analysis of insulin content in various Vigilon® patches**

| **Nominal dose** | **Insulin extracted** | **Insulin extracted** |
|---|---|---|
| (IU/Vigilon®) patch) | (IU/Vigilon® patch) | (% of initial dose) |
| 0.25 | 0.15 ± 0.04 | 58.83 ± 14.82 |
| 0.50 | 0.31 ± 0.08 | 61.40 ± 15.72 |
| 2.50 | 1.88 ± 0.13 | 75.19 ± 5.11 |
| 5.00 | 4.10 ± 0.45 | 81.19 ± 8.89 |

As shown in Table 4, the amount of insulin extracted from high dose patches (2.5 and 5 IU input) was 1.9 and 4.1 IU, which are 75 and 81 % of the initial dose applied to the patches, respectively. The amount of insulin extracted from low dose patches (0.25 and 0.5 IU input) was 0.15 and 0.31 IU, which are 59 and 61 % of the initial dose applied to the patches, respectively. The 20-40 % of the initial insulin input that were not extracted remained probably bound to the hydrogel. The non-extractable amount of insulin was higher when a low dose of insulin (0.25-0.5 IU/Vigilon® patch) was used probably because the binding of insulin to the hydrogel at such doses is higher.

### EXAMPLE 10

### In-vitro skin permeation study of insulin in insulin-Vigilon® patches

The permeability of insulin in Vigilon® patches through porcine skin was measured in vitro with a Franz diffusion cell system (home-made). The diffusion area was 2 cm². Dermatomized (300.500µm, Electric Dermatom, Padgett Instruments Ltd, Kansas, MI, USA) porcine skin was excised from slaughtered white pigs (breeding of Landres and Large White, Kibbutz Lahav, Israel). Transepidermal water loss measurements (TEWL, Dermalab Cortex Technology, Hadsund, Denmark) were performed and only skin pieces with TEWL levels less than 15g/m² /h were mounted in the diffusion cells.

Skin micro channeling was performed using the ViaDerm™ instrument as follows: The diameter of the microelectrode array used in all the studies was 80 µm, and the density was 75 microelectrodes/cm². The device was applied twice on each location, so the density of the micro channels was 150/cm². Rat skin was treated with an applied voltage of 330V, frequency of 100kHz, two bursts, 700 microsecond burst length, and no current limitation.

After ViaDerm application, the TEWL was measured again to control the operation. The skin pieces were placed on the acceptor cell with the stratum corneum facing upwards, and the donor chambers were clamped in place. The skin was washed 3 times with PBS, the PBS in the acceptor cells was replaced with PBS that contained 0.1 % sodium azide (Merk, Dermstadt, Germany), 1% bovine serum albumin (Biological Industries, Kibutz Beit-Hamek, Israel) and protease inhibitors cocktail (1 tablet per 50 ml PBS, Complete Mini, Roch), and then insulin-Vigilon® patches were placed on the stratum corneum side. Samples from the receiver solutions were collected into tubes at predetermined times for up to 12 hr period. The samples were kept at -20°C until analyzed. Insulin analysis was performed by ELIZA kit (DSL-10-1600, Diagnostic Systems Laboratories, Inc. Webster, Texas, USA).

### Results

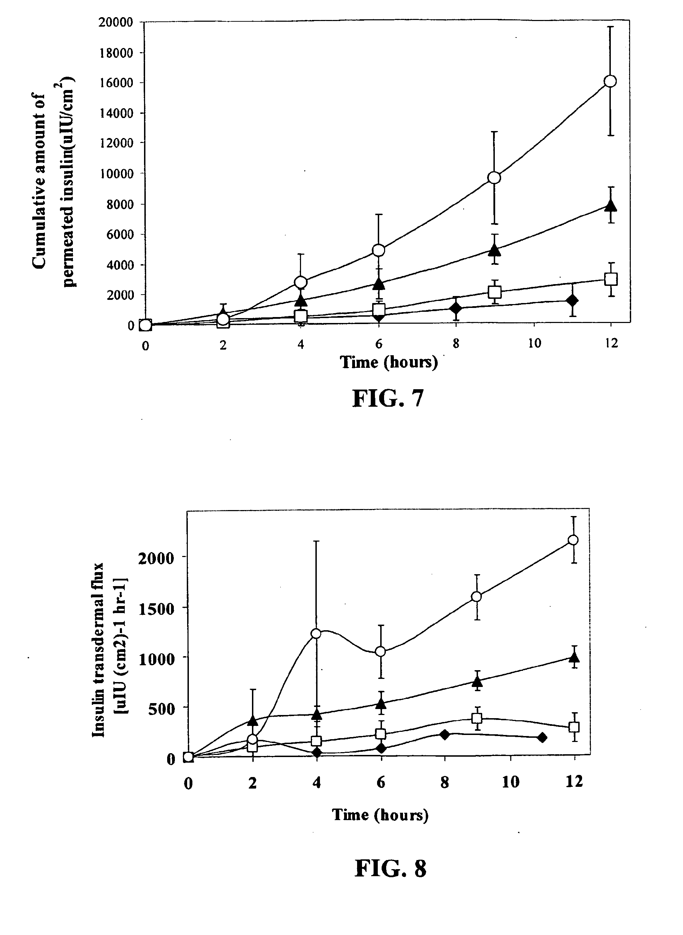

The cumulative permeability of insulin in Vigilon® patches through ViaDerm treated skin is shown in FIG. **7****.** After the administration of insulin, its cumulative amount increased as a function of time and the total permeation amount was dose dependent (1.5, 2.8, 7.8, and 16.0 mU/cm² after 12 hr for 0.25, 0.5, 2.5, and 5 IU insulin in Vigilon®, respectively, see FIG. **7**). The insulin transdermal flux values (amount permeated per cm² per hr) are shown in FIG. **8****.** The flux values also showed dose dependency. Thus, after 6 hr, for example, the values were 0.1, 0.2, 0.5, and 1.0 mUxcm²⁻¹xhr⁻¹ for 0.25, 0.5, 2.5, and 5 IU insulin in Vigilon®, respectively (see FIG. **8**).

The in-vitro results indicate that transdermal delivery of insulin can be achieved by its incorporation into a hydrogel and the use of the micro-channeling technology. The in-vitro permeation study suggests that sustained and controlled in-vivo insulin delivery may be achieved using the insulin- Vigilon® patches.

### EXAMPLE 11

### Bioactivity of insulin embedded in Vigilon® in diabetic rats

Male rats (300-325, Sprague Dawley, Harlan laboratories Ltd., Israel) were deprived of food and received water ad libitum 48 hr prior to patch applications. Streptozotocin (55 mg/kg in citric buffer, 0.1M, pH 4.5; Sigma, St. Louis, MO, USA) was injected IP to the rats 24 hr prior to patch applications in order to induce diabetes. The rats were defined as diabetic when 24 hrs after the injections the glucose levels were above 300 mg/dl, and positive urine glucose and negative urine ketones were observed. The diabetic rats were premedicated with IP injections of 10% ketamin/ 2% xylazine solution at a ratio of 70:30, 1 ml/kg. Anesthesia was maintained with either isofluorane or halothane gas. The abdominal skin hair was shaved carefully, and was cleaned with isopropyl alcohol. After 30 min, transepidermal water loss measurements (TEWL, Dermalab Cortex Technology, Hadsund, Denmark) were performed to check skin integrity. Skin micro channeling was performed by the use of the ViaDerm instrument with the conditions described herein above in Example 10. TEWL was then measured again to control the operation. The treated skin was covered with various insulin patches. All the rats received SC injections of 0.1 IU insulin two hours before the patch application. SC injections of 0.1 IU without patch application served as comparative controls. Blood was withdrawn from the rat's tail and glucose levels were determined using blood glucose test strips (Ascensia Elite, Bayer) at predetermined times post application.

### Results

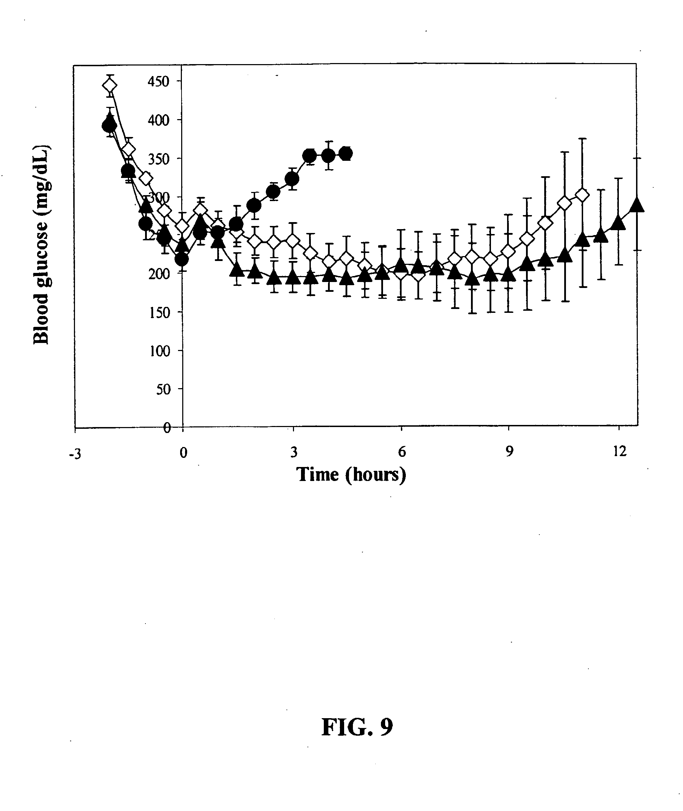

Blood glucose levels following the application of insulin-Vigilon® patches to micro-channeled skin of diabetic rats are shown in FIG. **9**. Subcutaneous administration of 0.1 IU insulin resulted in an immediate decrease in glucose levels (FIG. **9**). The SC effect continued for a short period of time and did not maintain a constant level of blood glucose. However, when insulin-Vigilon® patches were applied on the skin 2 hr after the SC injections, a controlled, constant, and sustained blood glucose profile was exhibited for 9 hr. No significant differences were observed between the 1.5 and 2.5 IU patches.

The present findings indicate that insulin-Vigilon® patches exhibit an extended delivery of insulin as reflected by sustained blood glucose profile. As a result, the biological effect of insulin was longer than that obtained after SC injection.

### EXAMPLE 12

### Preparation of hGH- or insulin-carrageenan film based patches

The following materials were used for the preparation of hGH- or insulin-carrageenan film based patches: Carrageenan Type I and type II (Sigma, MO, USA), human growth hormone (hGH) Genotropin^{®} (5.3mg/16 IU, Pharmacia and Upjohn, Stockholm, Sweden), human recombinant insulin Humulin^{®} R (Regular-100 IU/ml, Lilly France S.A., Fegershein, France), and phosphate buffered saline (PBS; Kibbutz Beit Haemak, Israel).

Carrageenan (2% w/v, type I and type II) was dissolved in ddH₂O at 45°C. The solution was cooled down to about 37°C, then a desired amount of either hGH or insulin was added from a concentrated stock solution, and the solution was mixed gently. The solution was air dried until a film was formed. The film was cut to a patch at dimensions of 1.4x1.4 cm. The patches were kept at 4°C until use.

Human GH-carrageenan films were prepared with initial loading of 9 µg hGH per 1 mg of carageenan. Typical film weighted approximately 10 mg, i.e., hGH weighted approximately 90 µg on each patch. Insulin-carrageenan films were prepared at doses of 1 and 5 IU per film.

### EXAMPLE 13

### In vitro release of hGH or insulin from carrageenan film based patches

For studying the release kinetics of hGH or insulin from carrageenan film based patches, the carrageenan films were placed in a PBS solution and incubated at room temperature. At different time points, the buffer was replaced with fresh buffer. The amount of hGH released to the buffer was determined by size exclusion HPLC, while the amount of insulin released to the buffer was analyzed by reverse phase HPLC.

### Results

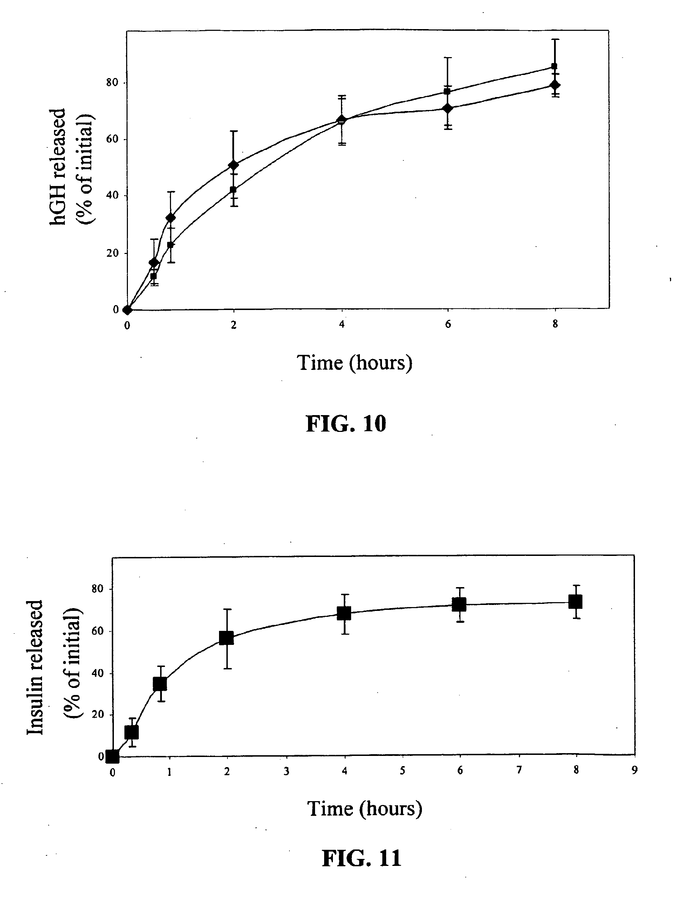

hGH release from type I and type II carrageenan films is shown in FIG. **10**. When type 1 carrageenan film was used, about 17% of the hGH initial amount was released after 20 min. hGH was released in a sustained manner over 8 hrs. The total recovered amount after 8 hrs was 80% of the initial dose. As the film started to dissolve after 8 hrs, further release was not measured.

When type II carrageenan film was used a sustained release profile was observed for 20 hr. About 12% of the initial amount of hGH applied to the film was released after 20 min, 85% were released after 8 hrs, and 91% of the initial amount was released after 20 hr.

Insulin release from type II carrageenan film is shown in FIG. **11**. Insulin was released from the carrageenan films in a sustained manner over 8 hrs. The total released amount after 8 hrs was about 70% of the initial amount applied to the film.

Thus, the in-vitro release of hGH and insulin from the carrageenan films showed a sustained profile.

### EXAMPLE 14

### In vitro skin permeation study of hGH and insulin from carrageenan based patches

The permeability of insulin through porcine skin was measured in vitro with a Franz diffusion cell system (house made). The diffusion area was 2 cm². Dermatomized (300-500µm, Electric Dermatom, Padgett Instruments Ltd, Kansas, MI, USA) porcine skin was excised from slaughtered white pigs (breeding of Landres and Large White, Kibbutz Lahav, Israel). Transepidermal water loss measurements (TEWL, Dermalab Cortex Technology, Hadsund, Denmark) were performed and only pieces with TEWL levels less than 15g/m² /h were mounted in the diffusion cells. Skin micro-channeling was performed using the ViaDerm™ instrument. The density of the microelectrodes was 200/cm². After ViaDerm application, the TEWL was measured again to control the operation. The skin pieces were washed 3 times with PBS, and insulin-carrageenan films were placed on the stratum corneum side and were covered with an adhesive. The skins plus the films were then placed on the acceptor cell with the stratum corneum facing upwards, and the donor chambers were clamped in place. The acceptor cells were filled with phosphate buffered saline (PBS, pH 7.4) that contained 0.1% sodium azide (Merk, Dermstadt, Germany), 1% bovine serum albumin (Biological Industries, Kibutz Beit-Hamek, Israel) and protease inhibitors cocktail (1 tablet per 50 ml PBS, Complete Mini, Roch). Samples from the receiver solutions were collected into tubes at predetermined times for up to 12 hr period. The samples were kept at 4°C until analyzed. Insulin analysis was performed by ELIZA kit (DSL-10-1600, Diagnostic Systems Laboratories, Inc. Webster, Texas, USA).

### Results

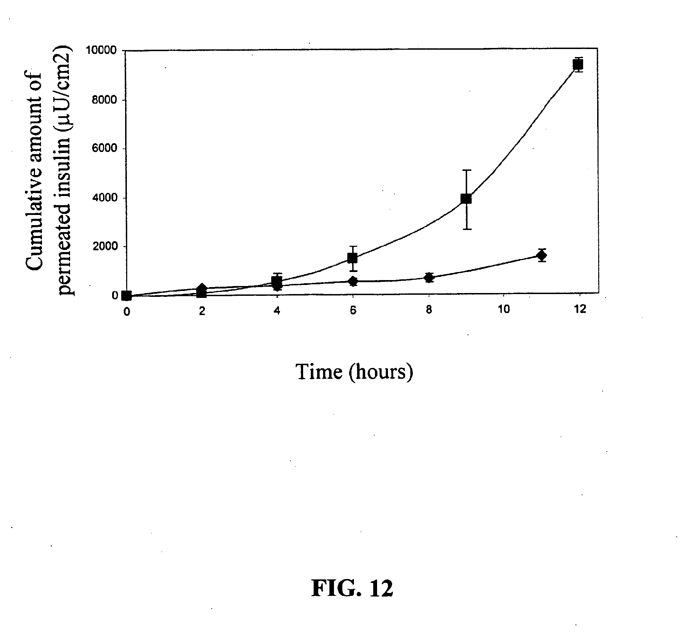

The cumulative permeability of insulin (1 and 4 IU) in carrageenan film-based patch through ViaDerm treated skin is shown in FIG. **12**. After administration of insulin in carrageenan film, the cumulative insulin amount increased as a function of time, and the total permeation amount was higher for 4IU patches than 1 IU patches (9.3 mU/cm² after 12 hr and 1.6 mU/cm² after 11 hr for 4 and 1 IU patches, respectively, FIG. **12**).

The initial loading of the protein in a carrageenan patch, carrageenan type and/or carrageenan amount can be adjusted to attain a desired extendable delivery in-vivo.

It will be appreciated by persons skilled in the art that the present invention is not limited by what has been particularly shown and described herein above. Rather the scope of the invention is defined by the claims that follow.

## Claims

1. A system for facilitating transdermal delivery of an active agent through skin of a subject comprising:
(i) an apparatus comprising:
a. an electrode cartridge comprising an array of electrodes; and
b. a main unit comprising a control unit, which is adapted to apply electrical energy to the electrodes when said electrodes are in vicinity of the skin, enabling ablation of stratum corneum in an area beneath the electrodes, thereby generating a plurality of micro-channels extending from the surface of the skin through all or a significant part of the stratum corneum; and
(ii) a patch adapted to be applied to the area of the skin where micro-channels are present after removal of the apparatus, the patch comprising at least one drug reservoir layer comprising a hydrophilic polymeric matrix and a pharmaceutical composition comprising as the active agent at least one hydrophilic therapeutic or immunogenic peptide, polypeptide, or protein, the at least one drug reservoir layer is formulated in a dry form or as a hydrogel.

2. The system according to claim 1,
wherein the electrode cartridge is adapted to generate a plurality of micro-channels of uniform shape and dimensions; or
wherein the electrical energy is of radio frequency.

3. The system according to claim 1, wherein the hydrophilic polymeric matrix is selected from the group consisting of hydrophilic biopolymers, hydrophilic synthetic polymers, derivatives and combinations thereof.

4. The system according to claim 3,
wherein the hydrophilic biopolymer is selected from the group consisting of hydroxypropyl cellulose, carboxymethyl cellulose, hydroxyethyl cellulose, carrageenans, chitin, chitosan, alginates, collagens, gelatin, pectin, glycosaminoglycans (GAGs), proteoglycans, fibronectins, and laminins, preferably wherein the biopolymer is selected from the group consisting of collagens and carrageenans; or
wherein the hydrophilic synthetic polymer is selected from the group consisting of polyglycolic acid (PGA), polylactic acid (PLA), polypropylene oxide, polyethylene oxide, polyoxyethylene-polyoxypropylene copolymers, polyvinylalcohol, polyethylene glycol, and polyurethanes, preferably, wherein the hydrophilic synthetic polymer is polyethylene oxide.

5. The system according to claim 1,
wherein the active agent is selected from growth factors, hormones, cytokines, water-soluble drugs, antigens, antibodies, fragments and analogs thereof; or
wherein the active agent is selected from the group consisting of insulin, proinsulin, follicle stimulating hormone, insulin like growth factor-1, insulin like growth factor-2, platelet derived growth factor, epidermal growth factor, fibroblast growth factors, nerve growth factor, transforming growth factors, tumor necrosis factor, calcitonin, parathyroid hormone, growth hormone, bone morphogenic protein, erythropoietin, hemopoietic growth factors, luteinizing hormone, glucagon, clotting factors, anti- clotting factors, atrial natriuretic factor, lung surfactant, plasminogen activators, bombesin, thrombin, enkephalinase, relaxin A-chain, relaxin B-chain, prorelaxin, inhibin, activin, vascular endothelial growth factor, hormone receptors, growth factor receptors, integrins, protein A, protein D, rheumatoid factors, neurotrophic factors, CD proteins, osteoinductive factors, immunotoxins, interferons, colony stimulating factors, interleukins (ILs), superoxide dismutase, surface membrane proteins, T-cell receptors, decay accelerating factor, viral antigens, transport proteins, homing receptors, addressins, regulatory proteins, analogs, derivatives and fragments thereof, preferably wherein the active agent is growth hormone or insulin; or
wherein the drug reservoir layer comprises a collagen and human growth hormone (hGH); or
wherein the drug reservoir layer comprises a collagen and human insulin; or
wherein the drug reservoir layer comprises polyethylene oxide and human growth hormone; or
wherein the drug reservoir layer comprises polyethylene oxide and human insulin; or wherein the drug reservoir layer comprises a carrageenan and human growth hormone; or
wherein the drug reservoir layer comprises a carrageenan and human insulin.

6. The system according to any of claims 1 to 5, wherein the patch further comprises at least one of the following layers: a backing layer, an adhesive, and a rate-controlling layer.

7. The system according to any of claims 1 to 6, wherein the pharmaceutical composition further comprises at least one component selected from the group consisting of protease inhibitors, stabilizers, anti-oxidants, buffering agents, and preservatives.

8. A system for use in sustained transdermal delivery of a hydrophilic therapeutic or immunogenic peptide, polypeptide or protein, the system comprises:
(i) an apparatus comprising:
(a) an electrode cartridge comprising an array of electrodes; and
(b) a main unit comprising a control unit, which is adapted to apply electrical energy to the electrodes when said electrodes are in vicinity of the skin, enabling ablation of stratum corneum in an area beneath the electrodes, thereby generating a plurality of micro-channels extending from the skin surface through all or a significant part of the stratum corneum; and
(ii) a patch adapted to be applied to the area of the skin where micro-channels are present after removal of the apparatus, the patch comprising at least one drug reservoir layer which comprises a hydrophilic polymeric matrix and a pharmaceutical composition comprising a hydrophilic therapeutic or immunogenic peptide, polypeptide, or protein, wherein the drug reservoir layer is formulated in a dry form or as a hydrogel.

9. A system for use in sustained transdermal delivery according to claim 8, wherein the hydrophilic polymeric matrix is selected from the group consisting of hydrophilic biopolymers, hydrophilic synthetic polymers, derivatives and combinations thereof.

10. A system for use according to claim 9,
wherein the hydrophilic biopolymer is selected from the group consisting of hydroxypropyl cellulose, carboxymethyl cellulose, hydroxyethyl cellulose, carrageenans, chitin, chitosan, alginates, collagens, gelatin, pectin, glycosaminoglycans (GAGs), proteoglycans, fibronectins, and laminins, preferably wherein the biopolymer is selected from the group consisting of collagens and carrageenans; or
wherein the hydrophilic synthetic polymer is selected from the group consisting of polypropylene oxide, polyethylene oxide, polyoxyethylene-polyoxypropylene copolymers, polyvinylalcohol, polyurethanes, preferably wherein the hydrophilic synthetic polymer is polyethylene oxide.

11. A system for use according to claim 8,
wherein the active agent is selected from the group consisting of growth factors, hormones, cytokines, water-soluble drugs, antigens, antibodies, fragments and analogs thereof; or
wherein the active therapeutic or immunogenic agent is selected from the group consisting of insulin, proinsulin, follicle stimulating hormone, insulin like growth factor-1, insulin like growth factor-2, platelet derived growth factor, epidermal growth factor, fibroblast growth factors, nerve growth factor, transforming growth factors, tumor necrosis factor, calcitonin, parathyroid hormone, growth hormone, bone morphogenic protein, erythropoietin, hemopoietic growth factors, luteinizing hormone, glucagon, clotting factors, anti- clotting factors, atrial natriuretic factor, lung surfactant, plasminogen activators, bombesin, thrombin, enkephalinase, relaxin A-chain, relaxin B-chain, prorelaxin, inhibin, activin, vascular endothelial growth factor, hormone receptors, growth factor receptors, integrins, protein A, protein D, rheumatoid factors, neurotrophic factors, CD proteins, osteoinductive factors, immunotoxins, interferons, colony stimulating factors, interleukins (ILs), superoxide dismutase, T-cell receptors, surface membrane proteins, decay accelerating factor, viral antigens, transport proteins, homing receptors, addressins, regulatory proteins, analogs, derivatives and fragments thereof, preferably wherein the therapeutic agent is growth hormone or insulin; or
wherein the drug reservoir layer comprises a collagen and human growth hormone; or
wherein the drug reservoir layer comprises a collagen and human insulin; or
wherein the drug reservoir layer comprises polyethylene oxide and human growth hormone; or
wherein the drug reservoir layer comprises polyethylene oxide and human insulin; or wherein the drug reservoir layer comprises carrageenan and human growth hormone; or wherein the drug reservoir layer comprises carrageenan and human insulin.

12. A system for use according to any of claims 8 to 11, wherein the patch further comprises at least one of the following layers: a backing layer, an adhesive, and a rate-controlling layer.

13. A system for use according to any of claims 8 to 12, wherein the pharmaceutical composition further comprises at least one component selected from the group consisting of protease inhibitors, stabilizers, anti-oxidants, buffering agents and preservatives.

## Patentansprüche

1. System, um eine transdermale Abgabe eines aktiven Wirkstoffs durch die Haut eines Individuums zu ermöglichen, umfassend:
(i) eine Vorrichtung, umfassend:
a. eine Elektrodenkassette, umfassend eine Anordnung von Elektroden; und
b. eine Haupteinheit, umfassend eine Steuereinheit, die angepasst ist die Elektroden mit elektrischer Energie zu versorgen, wenn sich die Elektroden in der Nähe der Haut befinden, ein Ablösen des Stratum Corneum in einem Gebiet unter den Elektroden zu ermöglichen, wodurch mehrere, sich von der Oberfläche der Haut durch das gesamte oder einen wesentlichen Teil des Stratum Corneum erstreckende Mikrokanäle erzeugt werden; und
(ii) einen Patch, der für ein Aufbringen auf das Hautbereich angepasst ist, in dem nach Entfernung der Vorrichtung Mikrokanäle vorliegen, wobei der Patch mindestens eine Arzneimittelvorratsschicht umfasst, die eine hydrophile Polymermatrix und eine pharmazeutische Zusammensetzung umfasst, die als den aktiven Wirkstoff mindestens ein hydrophile(s) therapeutische(s) oder immunogene(s) Peptid, Polypeptide, oder Protein umfasst, wobei die mindestens eine Arzneimittelvorratsschicht in einer trockenen Form oder als ein Hydrogel formuliert ist.

2. System nach Anspruch 1,
worin die Elektrodenkassette zum Erzeugen mehrerer Mikrokanäle einheitlicher Form und Abmessungen angepasst ist; oder
worin die elektrischer Energie aus Radiofrequenz besteht.

3. System nach Anspruch 1, worin die hydrophile Polymermatrix ausgewählt ist aus der Gruppe bestehend aus hydrophilen Biopolymeren, hydrophilen synthetischen Polymeren, Derivaten und Kombinationen davon.

4. System nach Anspruch 3,
worin das hydrophile Biopolymer ausgewählt ist aus der Gruppe bestehend aus Hydroxypropylcellulose, Carboxymethylcellulose, Hydroxyethylcellulose, Carrageenanen, Chitin, Chitosan, Alginaten, Collagenen, Gelatine, Pektin, Glycosaminoglycanen (GAGs), Proteoglycanen, Fibronektinen und Lamininen, wobei das Biopolymer vorzugsweise ausgewählt ist aus der Gruppe bestehend aus Collagenen und Carrageenanen; oder
worin das hydrophile synthetische Polymer ausgewählt ist aus der Gruppe bestehend aus Polyglycolsäure (PGA), Polymilchsäure (PLA), Polypropylenoxid, Polyethylenoxid, Polyoxyethylen-Polyoxypropylen Copolymeren, Polyvinylalkohol, Polyethylenglycol und Polyurethanen, wobei das hydrophile synthetische Polymer vorzugsweise Polyethylenoxid ist.

5. System nach Anspruch 1,
worin der aktive Wirkstoff ausgewählt ist aus Wachstumsfaktoren, Hormonen, Cytokinen, wasserlöslichen Arzneimitteln, Antigenen, Antikörpern, Fragmenten und Analogen davon; oder
worin der aktive Wirkstoff ausgewählt ist aus der Gruppe bestehend aus Insulin, Proinsulin, Follikel stimulierendes Hormon, Insulin ähnlicher Wachstumsfaktor-1, Insulin ähnlicher Wachstumsfaktor-2, Blutplättchenwachstumsfaktor, epidermaler Wachstumsfaktor, Fibroblastenwachstumsfaktoren, Nervenwachstumsfaktor, transformierender Wachstumsfaktoren, Tumornekrosefaktor, Kalcitonin, Parathyroidhormon, Wachstumshormon, Knochen morphogenes Protein, Erythropoietin, blutbildenden Wachstumsfaktoren, luteinisierendes Hormon, Glucagon, Gerinnungsfaktoren, anti-Gerinnungsfaktoren, atriales natriuretisches Hormon, Lungensurfaktant, Plasminogenaktivatoren, Bombesin, Thrombin, Enkephalinase, Relaxin A-Kette, Relaxin B-Kette, Prorelaxin, Inhibin, Activin, vaskulärer endothelialer Wachstumsfaktor, Hormonrezeptoren, Wachstumsfaktorrezeptoren, Integrinen, Protein A, Protein D, Rheumafaktoren, neurotrophen Faktoren, CD Proteine, osteoinduktiven Faktoren, Immunotoxinen, Interferonen, Kolonie stimulierenden Faktoren, Interleukinen (ILs), Superoxiddismutase, Oberfächenmembranproteinen, T-Zellrezeptoren, Verfall beschleunigender Faktor, viralen Antigenen, Transportproteinen, Homingrezeptoren, Addressinen, Regulatorproteinen, Analogen, Derivativen und Fragmenten davon, wobei der aktive Wirkstoff vorzugsweise Wachstumshormon oder Insulin ist; oder
worin die Arzneimittelvorratsschicht ein Kollagen und menschliches Wachstumshormon (hGH) umfasst; oder
worin die Arzneimittelvorratsschicht ein Collagen und menschliches Insulin umfasst; oder
worindie Arzneimittelvorratsschicht Polyethylenoxid und menschliches Wachstumshormon umfasst; oder
worin die Arzneimittelvorratsschicht Polyethylenoxid und menschliches Insulin umfasst; oder
worin die Arzneimittelvorratsschicht ein Carrageenan und menschliches Wachstumshormon umfasst; oder
worin die Arzneimittelvorratsschicht eine Carrageenan und menschliches Insulin umfasst.

6. System nach einem der Ansprüche 1 to 5, worin der Patch ferner mindestens eine nachstehend aufgeführten Schichten umfasst: eine Trägerschicht, einen Kleber und eine Raten-steuernde Schicht.

7. System nach einem der Ansprüche 1 to 6, worin die pharmazeutische Zusammensetzung ferner mindestens einen Bestandteil ausgewählt aus der Gruppe bestehend aus Proteaseinhibitoren, Stabilisatoren, anti-Oxidantien, Puffersubstanzen, und Konservierungsmitteln.

8. System zur Verwendung bei verzögerter transdermaler Abgabe eines hydrophilen therapeutischen oder immunogenen Peptids, Polypeptids oder Proteins, worin das System umfasst:
(i) eine Vorrichtung, umfassend:
(a) eine Elektrodenkassette umfassend eine Anordnung von Elektroden; und
(b) eine Haupteinheit umfassend eine Steuereinheit, die angepasst ist elektrischer die angepasst ist die Elektroden mit elektrischer Energie zu versorgen, wenn sich die Elektroden in der Nähe der Haut befinden, ein Ablösen des Stratum Corneum in einem Gebiet unter den Elektroden zu ermöglichen, wodurch mehrere, sich von der Oberfläche der Haut durch das gesamte oder einen bedeutenden Teil des Stratum Corneum erstreckende Mikrokanäle erzeugt werden; und
(ii) einen Patch, der für ein Aufbringen auf dem Hautgebiet angepasst ist, in dem Mikrokanäle nach Entfernung der Vorrichtung vorliegen, wobei der Patch mindestens eine Arzneimittelvorratsschicht umfasst, die eine hydrophile Polymermatrix und eine pharmazeutische Zusammensetzung umfasst, die als den aktiven Wirkstoff mindestens ein hydrophiles therapeutisches oder immunogene(s) Peptid, Polypeptide, oder Protein umfasst, wobei die mindestens eine Arzneimittelvorratsschicht in einer trockenen Form oder als ein Hydrogel formuliert ist.

9. System zur Verwendung bei verzögerter transdermaler Abgabe nach Anspruch 8, worin die hydrophile Polymermatrix ausgewählt ist aus der Gruppe bestehend aus hydrophilen Biopolymeren, hydrophilen synthetischen Polymeren, Derivaten und Kombinationen davon.

10. System zur Verwendung bei verzögerter transdermaler Abgabe nach Anspruch 9,
worin das hydrophile Biopolymer ausgewählt ist aus der Gruppe bestehend aus Hydroxypropylcellulose, Carboxymethylcellulose, Hydroxyethylcellulose, Carrageenanen, Chitin, Chitosan, Alginaten, Collagenen, Gelatine, Pektin, Glycosaminoglycanen (GAGs), Proteoglycanen, Fibronektinen, und Lamininen, wobei das Biopolymer vorzugsweise ausgewählt ist aus der Gruppe bestehend aus Collagenen und Carrageenanen; oder
worin das hydrophile synthetische Polymer ausgewählt ist aus der Gruppe bestehend aus Polypropylenoxid, Polyethylenoxid, Polyoxyethylen-Polyoxypropylen Copolymeren, Polyvinylalkohol, Polyurethanen, wobei das hydrophile synthetische Polymer vorzugsweise Polyethylenoxid ist.

11. System zur Verwendung bei verzögerter transdermaler Abgabe nach Anspruch 8,
worinder aktive Wirkstoff ausgewählt ist aus der Gruppe bestehend aus Wachstumsfaktoren, Hormonen, Cytokinen, wasserlöslichen Arzneimitteln, Antigenen, Antikörpern, Fragmenten und Analogen davon; oder
worin der aktive therapeutische oder immunogene Wirkstoff ausgewählt ist aus der Gruppe bestehend aus Insulin, Proinsulin, Follikel stimulierendes Hormon, Insulin ähnlicher Wachstumsfaktor-1, Insulin ähnlicher Wachstumsfaktor-2, Blutplättchenwachstumsfaktor, epidermaler Wachstumsfaktor, Fibroblastenwachstumsfaktoren, Nervenwachstumsfaktor, transformierender Wachstumsfaktoren, Tumornekrosefaktor, Kalcitonin, Parathyroidhormon, Wachstumshormon, Knochen morphogenes Protein, Erythropoietin, blutbildenden Wachstumsfaktoren, luteinisierendes Hormon, Glucagon, Gerinnungsfaktoren, anti-Gerinnungsfaktoren, atriales natriuretisches Hormon, Lungensurfaktant, Plasminogenaktivatoren, Bombesin, Thrombin, Enkephalinase, Relaxin A-Kette, Relaxin B-Kette, Prorelaxin, Inhibin, Activin, vaskulärer endothelialer Wachstumsfaktor, Hormonrezeptoren, Wachstumsfaktorrezeptoren, Integrinen, Protein A, Protein D, Rheumafaktoren, neurotrophen Faktoren, CD Proteine, osteoinduktiven Faktoren, Immunotoxinen, Interferonen, Kolonie stimulierenden Faktoren, Interleukinen (ILs), Superoxiddismutase, T-Zellrezeptoren, Oberfächenmembranproteinen, Verfall beschleunigender Faktor, viralen Antigenen, Transportproteinen, Homing Rezeptoren, Addressinen, Regulatorproteinen, Analogen, Derivaten und Fragmenten davon, wobei der aktive Wirkstoff vorzugsweise Wachstumshormon oder Insulin ist; oder
worin die Arzneimittelvorratsschicht ein Kollagen und menschliches Wachstumshormon umfasst; oder
worin die Arzneimittelvorratsschicht ein Collagen und menschliches Insulin umfasst; oder
wobei die Arzneimittelvorratsschicht Polyethylenoxid und menschliches Wachstumshormon umfasst; oder
worin die Arzneimittelvorratsschicht Polyethylenoxid und menschliches Insulin umfasst; oder
worin die Arzneimittelvorratsschicht ein Carrageenan und menschliches Wachstumshormon umfasst; oder
worin die Arzneimittelvorratsschicht eine Carrageenan und menschliches Insulin umfasst.

12. System zur Verwendung bei verzögerter transdermaler Abgabe nach einem der Ansprüche 8 to 11, worin der Patch ferner mindestens eine nachstehend aufgeführten Schichten umfasst: eine Trägerschicht, einen Kleber, und eine Raten-steuernde Schicht.

13. System zur Verwendung bei verzögerter transdermaler Abgabe nach einem der Ansprüche 8 to 12, worin die pharmazeutische Zusammensetzung ferner mindestens einen Bestandteil ausgewählt aus der Gruppe bestehend aus Proteaseinhibitoren, Stabilisatoren, anti-Oxidantien, Puffersubstanzen und Konservierungsmitteln.

## Revendications

1. Un système pour faciliter l'administration transdermique d'un agent actif au travers de la peau d'un sujet comprenant :
(i) un appareil comprenant :
(a) une cartouche d'électrode comprenant une série d'électrodes ; et
(b) une unité de contrôle principale adaptée pour appliquer de l'énergie électrique aux électrodes lorsque lesdites électrodes sont à proximité de la peau, permettant l'ablation du stratum corneum dans une zone située sous les électrodes, générant ainsi une pluralité de micro-canaux s'étendant à partir de la surface de la peau au travers de tout ou d'une portion importante du stratum corneum ; et
(ii) un patch adapté pour s'appliquer sur la zone de la peau où les micro-canaux sont présents après enlèvement de l'appareil, le patch comprenant au moins une couche réservoir de médicament comprenant une matrice polymère hydrophile et une composition pharmaceutique comprenant comme agent actif au moins un peptide, polypeptide ou protéine thérapeutique ou immunogène hydrophile, ladite au moins une couche réservoir de médicament étant formulé sous une forme sèche ou comme un hydrogel.

2. Le système selon la revendication 1 dans lequel la cartouche d'électrode est adaptée pour générer une pluralité de micro-canaux de forme et de dimensions uniformes ; ou dans lequel l'énergie électrique est une radiofréquence.

3. Le système selon la revendication 1 dans lequel la matrice polymère hydrophile est choisie à partir du groupe consistant en biopolymères hydrophiles, polymères synthétiques hydrophiles et combinaisons de ceux-ci.

4. Le système selon la revendication 3 dans lequel le biopolymère hydrophile est choisi à partir du groupe consistant en hydroxypropylcellulose, carboxyméthylcellulose, hydroxyéthylcellulose, carraghénanes, chitine, chitosane, collagènes, gélatine, pectine, glycosaminoglycanes (GAGs), protéoglycanes, fibronectines et laminines, de préférence dans lequel le biopolymère est choisi à partir du groupe consistant en collagènes et carraghénanes ; ou
dans lequel le polymère synthétique hydrophile est choisi à partir du groupe consistant en acide polyglycolique (PGA), acide polylactique (PLA), l'oxyde de propylène, l'oxyde de polyéthylène, copolymères de polyoxyéthyléne-polyoxypropylène, polyviniyl-alcool, polyéthylène glycol et polyuréthanes, de préférence dans lequel le polymère synthétique hydrophile est l'oxyde de polyéthylène.

5. Le système selon la revendication 1 dans lequel l'agent actif est choisi à partir de facteurs de croissance, hormones, cytokines, médicaments hydrosolubles, antigènes, anticorps, fragments et analogues de ceux-ci ; ou
dans lequel l'agent actif est choisi à partir du groupe consistant en insuline, pro insuline, hormone de stimulation des follicules, facteur de croissance-1 « insuline like », facteur de croissance-2 « insuline like », facteur de croissance dérivé de plaquettes, facteur de croissance épidermique, facteur de croissance des fibroblastes, facteur de croissance des nerfs, facteur de nécrose tumorale, calcitonine, hormone parathyroïdienne, hormone de croissance, protéine morphogénique osseuse, érythropoïétine, facteur de croissance hémopoïétique, hormone lutéinisante, glucagon, facteurs de coagulation, facteurs anticoagulants, facteur natriurétique auriculaire, surfactant pulmonaire, activateurs du plasminogène, bombésine, thrombine, encéphalinase, chaîne A de la relaxine, chaîne B de la relaxine, pro relaxine, inhibine, facteur de croissance endothélial vasculaire , récepteurs d'hormones, récepteurs du facteur de croissance, intégrines, protéine A, protéine D, facteurs rhumatoïdes, facteurs neurotrophiques, protéines CD , facteurs ostéo-inducteurs, immun toxines, interférons, facteurs de stimulations de colonies, interleukines (ILs), superoxyde dismutase, protéines de surface de membrane, récepteurs de cellules T, facteur d'accélération de carie, antigènes viraux, protéines de transport, récepteurs d'hébergement, se focalisant récepteurs, adressines, protéines de régulation, analogues, dérivés et fragments de ceux-ci, de préférence dans lequel l'agent actif est l'hormone de croissance ou l'insuline ; ou
dans lequel la couche réservoir de médicament comprend un collagène et l'hormone de croissance humaine (hGH) ; ou
dans lequel la couche réservoir de médicament comprend un collagène et l'insuline humaine ; ou
dans lequel la couche réservoir de médicament comprend de l'oxyde de polyéthylène et l'hormone de croissance humaine ; ou
dans lequel la couche réservoir de médicament comprend un carraghénane et l'hormone de croissance humaine ; ou
dans lequel la couche réservoir de médicament comprend un carraghénane et l'insuline humaine.

6. Le système selon l'une des revendications 1 à 5 dans lequel le patch comprend en outre au moins une des couches suivantes : une couche de fond, un adhésif et une couche de contrôle de vitesse.

7. Le système selon l'une des revendications 1 à 6 dans lequel la composition pharmaceutique comprend en outre au moins un composant choisi à partir du groupe consistant en inhibiteurs de protéase, stabilisateurs, antioxydants, agents tampons et conservateurs.

8. Un système pour usage dans l'administration transdermique soutenue d'un peptide, d'un polypeptide ou d'une protéine thérapeutique ou immunogénique hydrophile, le système comprenant :
(i) un appareil comprenant :
(a) une cartouche d'électrode comprenant une série d'électrodes ; et
(b) une unité de contrôle principale qui est adaptée pour appliquer de l'énergie électrique aux électrodes lorsque lesdites électrodes sont à proximité de la peau, permettant l'ablation du stratum corneum dans une zone située sous les électrodes, générant ainsi une pluralité de micro-canaux s'étendant à partir de la surface de la peau au travers de tout ou d'une portion importante du stratum corneum ; et
(ii) un patch adapté pour s'appliquer sur la zone de la peau où les micro-canaux sont présents après enlèvement de l'appareil, le patch comprenant au moins une couche réservoir de médicament comprenant une matrice polymère hydrophile et une composition pharmaceutique comprenant comme agent actif au moins un peptide, polypeptide ou protéine thérapeutique ou immunogène hydrophile, ladite au moins une couche réservoir de médicament étant formulé sous une forme sèche ou comme un hydrogel.

9. Un système pour usage dans l'administration transdermique soutenue selon la revendication 8 dans lequel la matrice polymère hydrophile est choisie à partir du groupe consistant en biopolymères hydrophiles, polymères synthétiques hydrophiles et combinaisons de ceux-ci.

10. Un système pour usage selon la revendication 9 dans lequelle biopolymère hydrophile est choisi à partir du groupe consistant en hydroxypropylcellulose, carboxyméthylcellulose, hydroxyéthylcellulose, carraghénanes, chitine, chitosane, collagènes, gélatine, pectine, glycosaminoglycanes (GAGs), protéoglycanes, fibronectines et laminines, de préférence dans lequel le biopolymère est choisi à partir du groupe consistant en collagènes et carraghénanes ; ou
dans lequel le polymère synthétique hydrophile est choisi à partir du groupe consistant en acide polyglycolique (PGA), acide ppolylactique (PLA), oxyde de propylène, oxyde de polyéthylène, copolymères de polyoxyéthhyléne-polyoxypropylène, polyviniyl-alcool, polyéthylène glycol et polyuréthanes, de préférence dans lequel le polymère synthétique hydrophile est l'oxyde de polyéthylène.

11. Un système pour usage selon la revendication 8 dans lequel l'agent actif est choisi à partir de facteurs de croissance, hormones, cytokines, médicaments hydrosolubles, antigènes, anticorps, fragments et analogues de ceux-ci ; ou
dans lequel l'agent actif est choisi à partir du groupe consistant en insuline, pro insuline, hormone de stimulation des follicules, facteur de croissance-1 « insuline like », facteur de croissance-2 « insuline like », facteur de croissance dérivé de plaquettes, facteur de croissance épidermique, facteur de croissance des fibroblastes, facteur de croissance des nerfs, facteur de nécrose tumorale, calcitonine, hormone parathyroïdienne, hormone de croissance, protéine morphogénique osseuse, érythropoïétine, facteur de croissance hémopoïétique, hormone lutéinisante, glucagon, facteurs de coagulation, facteurs anticoagulants, facteur natriurétique auriculaire, surfactant pulmonaire, activateurs du plasminogène, bombésine, thrombine, encéphalinase, chaîne A de la relaxine, chaîne B de la relaxine, pro relaxine, inhibine, facteur de croissance endothélial vasculaire , récepteurs d'hormones, récepteurs du facteur de croissance, intégrines, protéine A, protéine D, facteurs rhumatoïdes, facteurs neurotrophiques, protéines CD , facteurs ostéo-inducteurs, immun toxines, interférons, facteurs de stimulations de colonies, interleukines (ILs), superoxyde dismutase, protéines de surface de membrane, récepteurs de cellules T, facteur d'accélération de carie, antigènes viraux, protéines de transport, récepteurs d'hébergement, se focalisant récepteurs, adressines, protéines de régulation, analogues, dérivés et fragments de ceux-ci, de préférence dans lequel l'agent actif est l'hormone de croissance ou l'insuline ; ou
dans lequel la couche réservoir de médicament comprend un collagène et l'hormone de croissance humaine (hGH) ; ou
dans lequel la couche réservoir de médicament comprend un collagène et l'insuline humaine ; ou
dans lequel la couche réservoir de médicament comprend de l'oxyde de polyéthylène et l'hormone de croissance humaine ; ou
dans lequel la couche réservoir de médicament comprend un carraghénane et l'hormone de croissance humaine ; ou
dans lequel la couche réservoir de médicament comprend un carraghénane et l'insuline humaine.

12. Un système selon l'une des revendications 8 à 11 dans lequel le patch comprend en outre au moins une des couches suivantes : une couche de fond, un adhésif et une couche de contrôle de vitesse.

13. Un système pour usage selon l'une des revendications 8 à 12 dans lequel la composition pharmaceutique comprend en outre au moins un composant choisi à partir du groupe consistant en inhibiteurs de protéase, stabilisateurs, antioxydants, agents tampons et conservateurs.
